(19) Europäisches Patentamt
European Patent Office
Office européen des brevets

(11) EP 1 816 114 A1

(12) **EUROPEAN PATENT APPLICATION**
published in accordance with Art. 158(3) EPC

(43) Date of publication:
**08.08.2007 Bulletin 2007/32**

(21) Application number: **05809746.0**

(22) Date of filing: **25.11.2005**

(51) Int Cl.:
***C07C 15/38*** (2006.01) ***C07C 15/62*** (2006.01)
***C07C 25/22*** (2006.01) ***C07D 333/18*** (2006.01)
***C09K 11/06*** (2006.01) ***H01L 51/50*** (2006.01)
***C07C 22/08*** (2006.01)

(86) International application number:
**PCT/JP2005/021648**

(87) International publication number:
**WO 2006/057326 (01.06.2006 Gazette 2006/22)**

(84) Designated Contracting States:
**DE**

(30) Priority: **25.11.2004 JP 2004340362**
**06.09.2005 JP 2005257934**

(71) Applicants:
- **ROHM CO., LTD.**
**Kyoto-shi, Kyoto 615-8585 (JP)**
- **Pioneer Corporation**
**Tokyo 153-8654 (JP)**
- **HITACHI, LTD.**
**Chiyoda-ku**
**Tokyo 100-8280 (JP)**
- **Mitsubishi Chemical Corporation**
**Minato-ku**
**Tokyo 108-0014 (JP)**

(72) Inventors:
- **OYAMADA, Takahito**
**Saitama 3502201 (JP)**
- **UCHIUZOU, Hiroyuki**
**Kyushu University**
**Fukuoka 8128581 (JP)**
- **ADACHI, Chihaya**
**Kyushu University**
**Fukuoka 8128581 (JP)**
- **AKIYAMA, Seiji**
**Yokohama-shi, Kanagawa 2278502 (JP)**
- **TAKAHASHI, Takayoshi**
**Kyoto University**
**Kyoto 6158510 (JP)**

(74) Representative: **Grünecker, Kinkeldey, Stockmair & Schwanhäusser**
**Anwaltssozietät**
**Maximilianstrasse 58**
**80538 München (DE)**

(54) **PYRENE COMPOUND AND LIGHT EMITTING TRANSISTOR DEVICE UTILIZING THE SAME**

(57) An object is to provide a pyrene based compound that is good in both properties of light emission and mobility when the compound is used as a light emitting transistor element; and a light emitting transistor element wherein this specific pyrene based compound is used.

A pyrene based compound represented by the following chemical formula (1) is used as a main component of a light emitting layer in a light emitting transistor element:

[Chemical formula 15]

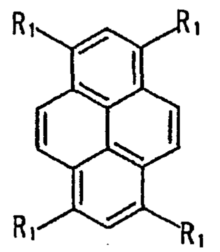

(1)

(wherein $R_1$ represents a group selected from a heteroaryl group which may have a substituent, an aryl group which may have a substituent except a phenyl group

EP 1 816 114 A1

which does not have any substituent, an alkyl group which may have a substituent and has a main chain having 1 to 20 carbon atoms, an alkenyl group which may have a substituent, an alkynyl group which may have a substituent, a silyl group which may have a substituent, and a halogen atom.)

## Description

TECHNICAL FIELD

The present invention relates to a pyrene based compound which becomes a main component of a light emitting layer in a light emitting transistor element, and a light emitting transistor element using the same.

BACKGROUND ART

Organic electroluminescence elements (hereinafter abbreviated to "organic EL elements"), which are typical examples of organic semiconductor devices, are light emitting elements using a light emitting phenomenon based on recombination of electrons and holes in a layer made of an organic fluorescent substance. Specifically, Patent Documents 1 and 2 and others describe organic EL elements each consisting of a light emitting layer made of the abovementioned organic compound, an electron injecting electrode for injecting electrons into this light emitting layer, and a hole injecting electrode for injecting holes into the light emitting layer.
Examples of the organic fluorescent substance used in this light emitting layer include perynone derivatives, distyrylbenzene derivatives (Patent Document 1), and 1,3,6,8-tetraphenylpyrene (Patent Document 2).
On the other hand, besides such organic EL elements, light emitting transistor elements are known as examples using a light emitting phenomenon based on recombination of electrons and holes in a layer made of an organic fluorescent substance. It is conceivable to use organic fluorescent substances as used in the abovementioned organic EL elements in such light emitting transistor elements.
Patent Document 1: JP-A-5-315078
Patent Document 2: JP-A-2001-118682

DISCLOSURE OF THE INVENTION

PROBLEMS TO BE SOLVED BY THE INVENTION

However, the molecules of compounds containing such pyrene based compounds are designed for organic EL elements, and substituents that hinder intermolecular interactions are introduced into the pyrene. For this reason, many of these compounds have a very high amorphousness.
However, when these compounds are used as light emitting transistor elements, it is necessary to design their molecules such that they are high in both light emission properties and mobility.
Thus, an object of the present invention is to provide a pyrene based compound that is good in both properties of light emission and mobility when the compound is used as a light emitting transistor element, and a light emitting transistor element using this specific pyrene based compound.

MEANS FOR SOLVING THE PROBLEMS

The present invention solves the above-mentioned problems by using a pyrene based compound represented by the following chemical formula (1) as a main component of a light emitting layer in a light emitting transistor element:

[Chemical formula 2]

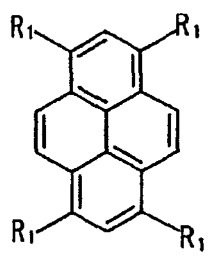

(1)

(wherein $R_1$ represents a group selected from a heteroaryl group which may have a substituent, an aryl group which may have a substituent (except a phenyl group which does not have any substituent), an alkyl group which may have a substituent and has a main chain having 1 to 20 carbon atoms, an alkenyl group which may have a substituent, an

alkynyl group which may have a substituent, a silyl group which may have a substituent, and a group having a halogen atom.)

A light emitting transistor element can be constructed by using the pyrene based compound as a main component of a light emitting layer which is capable of transporting holes and electrons as carriers and which emits light by recombination of the holes and the electrons, and by providing the light emitting layer with a hole injecting electrode for injecting holes into the light emitting layer, an electron injecting electrode for injecting electrons into the light emitting layer, and a gate electrode disposed opposite to the hole injecting electrode and the electron injecting electrode for controlling the carrier distribution in the light emitting layer.

EFFECTS OF THE INVENTION

According to the present invention, since a specific pyrene based compound having symmetry is used, the crystallinity improves, so that it is possible to improve both properties of the light emission and the mobility of the resultant light emitting transistor element.

BRIEF DESCRIPTION OF THE DRAWINGS

Fig. 1(a) is chemical formulae showing examples of the pyrene based compound.
Fig. 1(b) is chemical formulae showing examples of the pyrene based compound.
Fig. 1(c) is chemical formulae showing examples of the pyrene based compound.
Fig. 2(a) is chemical formulae showing examples of the pyrene based compound.
Fig. 2(b) is chemical formulae showing examples of the pyrene based compound.
Fig. 2(c) is chemical formulae showing examples of the pyrene based compound.
Fig. 2(d) is chemical formulae showing examples of the pyrene based compound.
Fig. 3(a) is chemical formulae showing examples of the pyrene based compound.
Fig. 3(b) is chemical formulae showing examples of the pyrene based compound.
Fig. 4(a) is chemical formulae showing examples of the pyrene based compound.
Fig. 4(b) is chemical formulae showing examples of the pyrene based compound.
Fig. 5 is a sectional view illustrating an example of the light emitting transistor element according to the present invention.
Fig. 6 is a plan view illustrating a structure of a source electrode and a drain electrode.
Figs. 7(a), (b) and (c) are schematic views illustrating the mechanism of light emission of a light emitting transistor element.
Fig. 8 is an electric circuit diagram illustrating an example of a display device wherein a light emitting transistor element according to the present invention is used.

DESCRIPTION OF REFERENCE NUMERALS OR SYMBOLS

| | |
|---|---|
| 1 | Light emitting layer |
| 2 | Source electrode |
| 2a | Comb tooth shaped region |
| 3 | Drain electrode |
| 3a | Comb tooth-shaped region |
| 4 | Gate electrode |
| 5 | Insulating film |
| 10 | Light emitting transistor element |
| 11 | Hole channel |
| 12 | Pinch-off point |
| 20 | Substrate |
| 21 | Display device |
| 22 | Scanning line driving device |
| 23 | Data line driving device |
| 24 | Controller |

| | |
|---|---|
| S | Source electrode |
| D | Drain electrode |
| G | Gate electrode |
| C | Capacitor |
| Ts | Selecting transistor |
| P11 & P12 | Pixels |
| LS1 & LS2 | Scanning lines |
| LD1 & LD2 | Data lines |

BEST MODE FOR EMBODYING THE INVENTION

The present invention will be described in detail hereinafter.
The present invention is an invention relating to a pyrene based compound, in particular, a pyrene based compound having symmetry. This pyrene based compound can be used as a main component of a light emitting layer in a light emitting transistor element.
The pyrene based compound is a compound represented by the following chemical formula (1):

[Chemical formula 3]

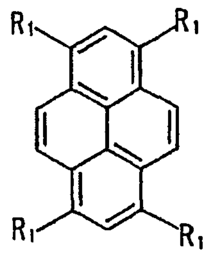

(1)

In the formula (1), $R_1$ represents a group selected from a heteroaryl group which may have a substituent, an aryl group which may have a substituent (except a phenyl group which does not have any substituent), an alkyl group which may have a substituent and has a main chain having 1 to 20 carbon atoms, an alkenyl group which may have a substituent, an alkynyl group which may have a substituent, a silyl group which may have a substituent, and a group having a halogen atom.
Specific examples of $R_1$ include heteroaryl, aryl, linear or branched alkyl, alkenyl, alkynyl and silyl groups, and groups having a halogen atom.
Specific examples of the heteroaryl group include benzofuryl, pyrrolyl, benzoxazolyl, pyrazinyl, thienyl, alkyl-substituted thienyl, bithienyl, phenyl-thienyl, benzothienyl, pyridyl, bipyridyl, phenyl-pyridyl, quinolyl, and benzothiazolyl groups. They may have a substituent. This heteroaryl group may be a polycyclic aromatic group.
Specific examples of the aryl group include naphthyl (preferably 2-naphthyl), anthryl (preferably 2-anthryl), phenanthryl, methylphenyl, ethylphenyl, dimethylphenyl, biphenyl, terphenyl, phenyl-etheno-phenyl, pyridino-phenyl, and fluorine-substituted phenyl groups. These may have a substituent. This aryl group may be a polycyclic aromatic group, but is not a phenyl group having no substituent.
Specific examples of the linear or branched alkyl group include methyl, ethyl, n-propyl, 2-propyl, n-butyl, isobutyl, and tert-butyl groups. The main chain of this alkyl group preferably has 1 to 20 carbon atoms.
Specific examples of the alkenyl group include vinyl, phenyl-substituted vinyl, ethyl-substituted vinyl, biphenyl-substituted vinyl, allyl, and 1-butenyl groups. They may have a substituent.
Specific examples of the alkynyl group include ethynyl, phenyl-substituted ethynyl, trimethylsilyl-substituted ethynyl, and propargyl groups. They may have a substituent.
Specific examples of the silyl group include a trimethylsilyl group. The group may have a substituent.
Specific examples of the group having a halogen atom include fluorine, bromine, and chlorine atoms. Of these groups, groups each consisting only of a halogen atom are preferable, and a fluorine atom is more preferable.
$R_1$ is preferably a group selected from benzofuryl, pyrrolyl, benzoxazolyl, pyrazinyl, thienyl, pyridyl, quinolyl, benzothiazolyl, naphthyl, anthryl, phenanthryl, vinyl, ethynyl and silyl groups each of which may have a substituent, a

phenyl group which has a substituent, a carboxyl group, and a halogen atom.

Particularly preferably, $R_1$ is a group selected from carboxyl, benzofuryl, pyrrolyl, benzoxazolyl, pyrazinyl, thienyl, alkyl-substituted thienyl, bithienyl, phenyl-thienyl, benzothienyl, pyridyl, bipyridyl, phenyl-pyridyl, quinolyl, benzothiazolyl, 2-naphthyl, 2-anthryl, phenanthryl, methylphenyl, ethylphenyl, dimethylphenyl, phenyl-substituted vinyl, phenyl-substituted ethynyl, biphenyl, terphenyl, phenyl-etheno-phenyl, pyridine-phenyl, fluorine-substituted phenyl, ethyl-substituted vinyl, biphenyl-substituted vinyl, trimethylsilyl and trimethylsilyl-substituted ethynyl groups, and a fluorine atom.

The molecular weight of the pyrene based compound is preferably 300 or more, more preferably 500 or more, and is preferably 5000 or less, more preferably 3000 or less.

Specific examples of the chemical formula (1) include compounds as shown in Fig. 1(a) to Fig. 3(b). Specifically, examples of the compound wherein $R_1$ is a heterocycle (heteroaryl) which may have a substituent include pyrene based compounds wherein $R_1$ is a thiophene ring (thienyl group) ((2-1) and (2-2) in Fig. 1(a)), a pyrene based compound wherein $R_1$ is a bithiophene ring (bithienyl group) ((2-3)) in Fig. 1(a)), a pyrene based compound wherein $R_1$ is a phenylthiophene ring (phenyl-thienyl group) ((2-4) in Fig. 1(a)), a pyrene based compound wherein $R_1$ is a benzothiophene ring (benzothienyl group) ((2-5) in Fig. 1(a)), pyrene based compounds wherein $R_1$ is a pyridine ring (pyridyl group) ((2-6) to (2-8) in Fig. 1(a)), a pyrene based compound wherein $R_1$ is a bipyridine ring (bipyridyl group) ((2-9) in Fig. 1 (b)), a pyrene based compound wherein $R_1$ is a phenylpyridine ring (phenyl-pyridyl group) ((2-10) in Fig. 1(b)), a pyrene based compound wherein $R_1$ is a quinoline ring (quinolyl group) ((2-11) in Fig. 1(b)), a pyrene based compound wherein $R_1$ is a benzothiazole ring (benzothiazolyl group) ((2-12) in Fig. 1(b)), a pyrene based compound wherein $R_1$ is a hexyl-substituted thiophene ring (thienyl group) ((2-13) in Fig. 1(e)), a pyrene based compound wherein $R_1$ is a hexyl-substituted bithiophene ring (bithienyl group) ((2-14) in Fig. 1(c)), and a pyrene based compound wherein $R_1$ is a benzoxazol ring (benzoxazolyl group) ((2-15) in Fig. 1(c)).

Examples of the compound wherein $R_1$ is an aryl group which may have a substituent, an alkenyl group which may have a substituent, or an alkynyl group which may have a substituent include pyrene based compounds wherein $R_1$ is a tolyl group ((3-1) to (3-2) in Fig. 2(a)), pyrene based compounds wherein $R_1$ is a dimethylphenyl group ((3-3) to (3-4) in Fig. 2(a)), a pyrene based compound wherein $R^1$ is a phenyl-substituted vinyl group ((3-5) in Fig. 2(a)), a pyrene based compound wherein $R_1$ is a phenyl-substituted vinyl group ethynyl group ((3-6) in Fig. 2(a)), pyrene based compounds wherein $R_1$ is a biphenyl group ((3-7) to (3-8) in Fig. 2(b)), a pyrene based compound wherein $R_1$ is a phenyl-etheno-phenyl group ((3-9) in Fig. 2(b)), a pyrene based compound wherein $R_1$ is a pyridine-phenyl group ((3-10) in Fig. 2(b)), a pyrene based compound wherein $R_1$ is a fluorine-substituted phenyl group ((3-11) in Fig. 2(b)), and ((3-12) to (3-15) in Fig. 2(c)), a pyrene based compound wherein $R_1$ is a terphenyl group ((3-16) in Fig. 2(d)), and a pyrene based compound wherein $R_1$ is a biphenyl-substituted vinyl group ((3-17) in Fig. 2(d)),

Examples of the compound wherein $R_1$ is an alkyl group which may have a substituent and has a main chain having 1 to 20 carbon atoms, an aryl group which may have a substituent, a silyl group which may have a substituent, or a fluorine atom include a pyrene based compound wherein $R_1$ is a phenanthrene ring (phenanthryl group) ((4-1) in Fig. 3(a)), a pyrene based compound wherein $R_1$ is a 2-naphthyl group ((4-2) in Fig. 3(a)), a pyrene based compound wherein $R_1$ is a 2-anthryl group ((4-3) in Fig. 3(a)), a pyrene based compound wherein $R_1$ is an ethyl-substituted vinyl group ((4-4) in Fig. 3(a)), a pyrene based compound wherein $R_1$ is a trimethylsilyl group ((4-5) in Fig. 3(a), wherein Me represents a methyl group), a pyrene based compound wherein $R_1$ is a trimethylsilylethynyl group ((4-6) in Fig. 3(b), wherein Me represents a methyl group), and a pyrene based compound wherein $R_1$ is a fluorine atom ((4-7) in Fig. 3(b)).

Furthermore, other examples of the pyrene based compound according to the present invention include individual compounds shown as (4-1) and (4-19) in Figs. 4(a) and (b).

Of the above-mentioned individual compounds, a pyrene based compound wherein $R_1$ is a group having a halogen atom is a compound which has not been known in the prior art.

The above-mentioned light emitting layer contains, as a main component thereof, the above-mentioned pyrene based compound. This main component means a component which takes a leading part for exhibiting luminous brightness, luminous efficiency, carrier mobility, peculiar light color, and other effects. In order to improve the above-mentioned effects, the light emitting layer may contain, besides the pyrene based compound as the main component, a secondary constituting component such as a different organic fluorescent substance or a dopant material if necessary.

Such a different organic fluorescent substance is not particularly limited, and examples thereof include condensed ring derivatives such as anthracene, phenanthrene, pyrene, perylene and chrysene, metal complexes of a quinolinol derivatives, such as tris(8-quinolinolato) aluminum, benzoxazole derivatives, stilbene derivatives, benzthiazole derivatives, thiadiazole derivatives, thiophene derivatives, tetraphenylbutadiene derivatives, cyclopentadiene derivatives, oxadiazole derivatives, bis-styryl derivatives such as bis-styryl anthracene and distyrylbenzene derivatives, metal complexes wherein a quinolinol derivative is combined with a different ligand, oxadiazole derivative metal complexes, benzazole derivative metal complexes, coumarin derivatives, pyrrolopyridine derivatives, perynone derivatives, and thiadiazolopyridine derivatives. Other examples of the organic fluorescent substance of a polymeric type include polyphenylene vinylene derivatives, polyparaphenylene derivatives, and polythiophene derivatives.

The above-mentioned dopant material is not particularly limited, and examples thereof include condensed ring derivatives such as phenanthrene, anthracene, pyrene, tetracene, pentacene, perylene, naphthopyrene, dibenzopyrene and rubrene, benzoxazole derivatives, benzthiazole derivatives, benzimidazole derivatives, benztriazole derivatives, oxazole derivatives, oxadiazole derivatives, thiazole derivatives, imidazole derivatives, thiadiazole derivatives, triazole derivatives, pyrazoline derivatives, stilbene derivatives, thiophene derivatives, tetraphenylbutadiene derivatives, cyclopentadiene derivatives, bis-styryl derivatives such as bis-styryl anthracene derivatives and distyrylbenzene derivatives, diazaindacene derivatives, furan derivatives, benzofuran derivatives, isobenzofuran derivatives such as phenylisobenzofuran, dimesitylisobenzofuran, di(2-methylphenyl) isobenzofuran, di(2-trifluoromethylphenyl)isobenzofuran and phenylisobenzofuran, dibenzofuran derivatives, coumarin derivatives such as 7-dialkylaminocoumarin derivatives, 7-piperidinocoumarin derivatives, 7-hydroxycoumarin derivatives, 7-methoxycoumarin derivatives, 7-acetoxycoumarin derivatives, 3-benzthiazolylcoumarin derivatives, 3-benzimidazolylcoumarin derivatives and 3-benzoxazolylcoumarin derivatives, dicyanomethylenepyran derivatives, dicyanomethylene-thiopyran derivatives, polymethine derivatives, cyanine derivatives, oxobenzanthracene derivatives, xanthene derivatives, rhodamine derivatives, fluorescein derivatives, pyrylium derivatives, carbostyril derivatives, acridine derivatives, bis(styryl)benzene derivatives, oxazine derivatives, phenylene oxide derivatives, quinacridone derivatives, quinazoline derivatives, pyrrolopyridine derivatives, furopyridine derivatives, 1,2,5-thiadiazolopyrene derivatives, perynone derivatives, pyrrolopyrrole derivatives, squalirium derivatives, violanthrone derivatives, phenazine derivatives, acridone derivatives, and diaza-flavin derivatives.

Description is now made of a light emitting transistor element using the above-mentioned pyrene based compound.

The light emitting transistor element may be an element having a basic structure of a field effect transistor (FET) as illustrated in Fig. 5.

This light emitting transistor element 10 comprises a light emitting layer 1 which is capable of transporting holes and electrons as carriers, which emits light by recombination of the holes and the electrons, and which contains the above-mentioned pyrene based compound as a main component; a hole injecting electrode for injecting holes into this light emitting layer 1, i.e., what is called a source electrode 2; an electron injecting electrode for injecting electrons into the light emitting layer, i.e., what is called a drain electrode 3; and a gate electrode 4 which is provided opposite to the source electrode 2 and the drain electrode 3 and is made of an $N^+$ silicon substrate to control the distribution of the carriers in the light emitting layer 1. The gate electrode 4 may be made of an electroconductive layer comprising an impurity diffusion layer formed on the surface of the silicon substrate.

Specifically, as shown in Fig. 5, an insulating film 5 made of silicon oxide or the like is formed on the gate electrode 4, and the source electrode 2 and the drain electrode 3 are formed thereon at an interval. The light emitting layer 1 is formed to cover the source electrode 2 and the drain electrode 3 and to be disposed between the two electrodes.

In order for the above-mentioned element to exhibit the function of the light emitting transistor, it is preferred that the difference between the HOMO energy level and the LUMO energy level of the organic fluorescent substance which constitutes the light emitting layer 1, in particular, the pyrene based compound as the main component thereof, the carrier mobility thereof, or the luminous efficiency thereof satisfies a predetermined range. When the pyrene based compound having the above-mentioned individual characteristics is used, it is possible to improve the individual functions by adding the above-mentioned secondary constituting component, such as the dopant, thereto.

First, the difference between the HOMO energy level and the LUMO energy level is preferably as small as possible so that the electrons can move more easily, and thus the light emission and the semi-conductivity (that is, the conductivity of electrons or holes in one direction) can be generated more easily. Specifically, the difference is preferably 5 eV or less, more preferably 3 eV or less, even more preferably 2.7 eV or less. Because the smaller this difference, the better the results, the lower limit of this difference is 0 eV.

The carrier mobility is preferably as high as possible for improved semi-conductivity. Specifically, the carrier mobility is preferably $1.0 \times 10^{-5}$ $cm^2$/V.s or more, more preferably $3.0 \times 10^{-5}$ $cm^2$/V.s or more, even more preferably $1.0 \times 10^{-4}$ $cm^2$/V.s or more. The upper limit of the carrier mobility is not particularly limited, and it is sufficient if the upper limit is about 1 $cm^2$/V.s.

The above-mentioned luminous efficiency means the ratio of light generated by the injection of photons or electrons. The ratio of emitted optical energy to injected optical energy is defined as the PL luminous efficiency (or PL quantum efficiency), and the ratio of the number of emitted photons to the number of injected electrons is defined as the EL luminous efficiency (or the EL quantum efficiency).

Injected and excited electrons emit light by recombining with holes. This recombination does not necessarily occur with a probability of 100%. Therefore, when organic compounds which each constitute the light emitting layer 1 are compared with each other, the EL luminous efficiencies are compared, thereby making it possible to compare the ratios of the emitted optical energy amount to injected optical energy, and compare synergetic effects about the ratio of the recombination of electrons and holes. Incidentally, by comparing the PL luminous efficiencies, the ratios of the emitted optical energy amount to injected optical energy can be compared. Thus, by comparing both the PL luminous efficiencies and the EL luminous efficiencies and combining the results, it is possible to compare the ratios of the recombination of

electrons and holes.

For the PL luminous efficiency, the degree of light emission is preferably as high as possible. The PL luminous efficiency is preferably 20% or more, more preferably 30% or more. The upper limit of the PL luminous efficiency is 100%.

For the EL luminous efficiency, the degree of light emission is preferably as high as possible. The EL luminous efficiency is preferably $1 \times 10\text{-}^{3}$% or more, more preferably $8 \times 10^{-3}$% or more. The upper limit of the EL luminous efficiency is 100%.

The light emitting transistor element 10 is characterized by the wavelength of emitted light besides the above. This wavelength is in a visible ray range. The element has a wavelength varied in accordance with the kind of the organic fluorescent substance used, in particular, the pyrene based compound. When organic fluorescent substances having different wavelengths are combined with each other, various colors can be produced. Fur this reason, about the wavelength of emitted light, the wavelength itself exhibits a characteristic.

The light emitting transistor element 10 is characterized by light emission. Thus, the element preferably has a luminous brightness to a certain extent. This luminous brightness is defined as the light emission amount corresponding to the brightness of an object felt by a person when the person watches the object. This luminous brightness is preferably as high as possible when measured by a photo-counter. The luminous brightness is preferably $1 \times 10^4$ CPS (count per sec) or more, more preferably $1 \times 10^5$ CPS or more, even more preferably $1 \times 10^6$ CPS or more.

The light emitting layer 1 is formed by depositing an organic fluorescent substance or the like that constitute the light emitting layer 1 (or co-depositing a plurality of such substances). It is sufficient if the film thickness of this light emitting layer is at least about 70 nm.

The source electrode 2 and the drain electrode 3 are electrodes for injecting holes and electrons into the light emitting layer 1, and are made of gold (Au), magnesium-gold alloy (MgAu), or the like. The electrodes are formed so as to face each other at a very small interval of, for example, 0.4 to 50 μm. Specifically, for example, as shown in Fig. 6, the source electrode 2 and the drain electrode 3 are formed to have comb tooth shaped regions 2a and 3a, respectively, which are each made of a plurality of comb teeth. The comb teeth which constitute the comb tooth shaped region 2a of the source electrode 2 and the comb teeth which constitute the comb tooth shaped region 3a of the drain electrode 3 are alternately arranged at predetermined intervals, whereby the light emitting transistor element 10 can exhibit the function thereof more effectively.

At this time, the interval between the source electrode 2 and the drain electrode 3, that is, the interval between the comb tooth shaped region 2a and the comb tooth shaped region 3a is preferably 50 μm or less, more preferably 3 μm or less, even more preferably 1 μm or less. If the interval is more than 50 μm, sufficient semi-conductivity cannot be exhibited.

By applying a voltage to the source electrode 2 and the drain electrode 3 in the light emitting transistor element 10, holes and electrons are shifted inside the element and they are recombined in the light emitting layer 1, whereby light can be emitted. At this time, the amounts of the holes and the electrons shifted between the two electrodes across the light emitting layer 1 depend on the voltage applied to the gate electrode 4. Accordingly, by controlling the voltage applied to the gate electrode 4 and its change, it is possible to control the state of electric conduction between the source electrode 2 and the drain electrode 3. Because this light emitting transistor element 10 undergoes P-type driving, a negative voltage for the source electrode 2 is applied to the drain electrode 3 and a negative voltage for the source electrode 2 is applied to the gate electrode 4.

Specifically, by applying a negative voltage for the source electrode 2 to the gate electrode 4, holes in the light emitting layer 1 are attracted toward the gate electrode 4, so that the density of holes in the vicinity of the surface of the insulating film 5 increases. By suitably adjusting the voltage between the source electrode 2 and the drain electrode 3, holes are injected from the source electrode 2 into the light emitting layer 1 according to the intensity of the controlled voltage applied to the gate electrode 4, so that electrons are injected from the drain electrode 3 into the light emitting layer 1. In other words, the source electrode 2 functions as a hole injecting electrode, and the drain electrode 3 functions as an electron injecting electrode. In this way, in the light emitting layer 1, the holes and the electrons are recombined, and light is emitted following this recombination. This light emission state can be turned on or off or the luminous intensity can be varied by changing the controlled voltage applied to the gate electrode 4.

The theory of such recombination of holes and electrons can be described as follows:

When a negative voltage for the source electrode 2 is applied to the gate electrode 4, in the light emitting layer 1, as illustrated in Fig. 7(a), channel 11 of holes are formed near the interface of the insulating film 2 so that a pinch-off point 12 thereof forms in the vicinity of the drain electrode 3. A high electric field is then formed between the pinch-off point 12 and the drain electrode 3, so that as shown in Fig. 7(b), the energy band is significantly bent. This produces an FN (Fowler-Nordheim) tunnel effect in which electrons in the drain electrode 3 penetrate through the potential barrier between the drain electrode 3 and the light emitting layer 1, so that the electrons are injected into the light emitting layer 1 and recombined with the holes.

The recombination of holes and electrons can also be described on the basis of the following theory besides the FN tunnel effect. As shown in Fig. 7(c), electrons at the HOMO energy level of the organic fluorescent substance in the light emitting layer 1 are excited to the LUMO level thereof by a high electric field. The excited electrons are recombined with holes in the light emitting layer 1. At the same time, electrons are injected from the drain electrode 3 to the HOMO energy level, which is now empty due to the excitation to the LUMO energy level, so that the empty level is filled.

A plurality of such light emitting transistor elements 10 are two-dimensionally arranged on a substrate 20 to form a display device 21. Fig. 8 shows an electric circuit diagram of this display device 21. Specifically, in this display device 21, light emitting transistor elements 10 as described above are each arranged in one of pixels P11, P12, ...,..., P21, P22, ...,..., which are arranged in a matrix form. The light emitting transistor elements 10 in these pixels are selectively caused to emit light and further the luminous intensity (brightness) of the light emitting transistor element 10 in each of the pixels is controlled, whereby two-dimensional display can be attained. The substrate 20 may be, for example, a silicon substrate integrated with the gate electrode 4. In other words, the gate electrode 4 may be made of an electroconductive layer which is an impurity diffusion layer wherein a pattern is formed in a surface of a silicon substrate. As the substrate 20, a glass substrate may be used.

Since each of the light emitting transistor elements 10 undergoes P-type driving, a bias voltage $V_d$ (< 0) is given to its drain electrode 3(D) with the source electrode 2(S) kept at the ground voltage (= 0). To its gate electrode 4(G), a selecting transistor Ts for selecting a pixel and a capacitor C for storing data are connected in parallel.

The selecting transistors Ts in each row of the pixels P11, P12, ....,..., P21, P22, ...,..., have their gates connected to a common one of the scanning line LS1, LS2, ,...,.... The selecting transistors Ts in each column of the pixels P11, P21, ...,..., P12, P22, ...,... are connected to a common one of the data lines LD1, LD2.... on their side opposite to the respective light emitting transistor elements 10.

From a scanning line driving circuit 22 controlled by a controller 24, scanning driving signals for selecting the pixels P11, P12, ...,...,P21, P22, ...,... in the respective rows circularly and successively (selecting the plurality of pixels in each row at a time) are given to the scanning lines LS1, LS2, ...,...In other words, the scanning line driving circuit 22 makes it possible to specify each of the rows successively as a selected row and make the selecting transistors Ts of the plurality of pixels in the selected row electrically conductive at a time, thereby generating a scanning driving signal for cutting off the selecting transistors Ts of the plurality of pixels in the non-selected rows at a time.

On the other hand, signals from a data line driving circuit 23 are inputted into the data lines LD1, LD2, ...,.... Control signals corresponding to image data are inputted from the controller 24 into this data line driving circuit 23. At a timing when the pixels in each of the rows are collectively selected by the scanning line driving circuit 21, the data line driving circuit 23 supplies light emission controlling signals, which correspond to the light emission gradations of the individual pixels in the selected row, to the data lines LD1, LD2, ...,.. in parallel.

In this way, in the individual pixels in the selected row, the light emission controlling signals are given to the gate electrodes 4(G) through the selecting transistors Ts. Thus, the light emitting transistor elements 10 in the pixels emit light having gradations corresponding to the light emission controlling signals (or stop the light emission). Since the light emission controlling signals are kept in the capacitor C, the electric potentials of the gate electrodes 4(G) are kept even after the selected row selected by the scanning line driving circuit 22 is shifted to a different row. As a result, the light emission states of the light emitting transistor elements 10 are kept.
Thus, two-dimensional display can be attained.

## EXAMPLES

The present invention will be more specifically described by way of examples and comparative examples described below. First, the process for producing the pyrene based compound will be described.

(Production Example 1) Production of tetrakis(2-thienyl)pyrene

[Synthesis of raw material] (Production of 1,3,6,8-tetrabromopyrene)

To 195 ml of water, 27 g of pyrene (reagent made by Tokyo Kasei Kogyo Co., Ltd., purity: 95%) and 7 ml of tetraglyme (reagent made by Tokyo Kasei Kogyo Co., Ltd.) were added, and 70 ml of hydrochloric acid was further added thereto. The mixture was stirred at 90°C for 2 hours to prepare an aqueous dispersion of pyrene. Next, 47 g of potassium bromide (reagent made by Tokyo Kasei Kogyo Co., Ltd.) was added thereto at 40°C. While the temperature was kept, a solution of sodium bromate in which 30 g of sodium bromate (reagent made by Tokyo Kasei Kogyo Co., Ltd.) was dissolved in 110 ml of water was dropwise added to the dispersion for 3 hours. Thereafter, the resultant was filtered, washed sufficiently with about 300 g of methanol, and dried at 85 to 95°C to yield 70 g of 1,3,6,8-tetrabromopyrene.

[Production of tetrakis(2-thienyl)pyrene (chemical formula (2-1))]

[Chemical formula 4]

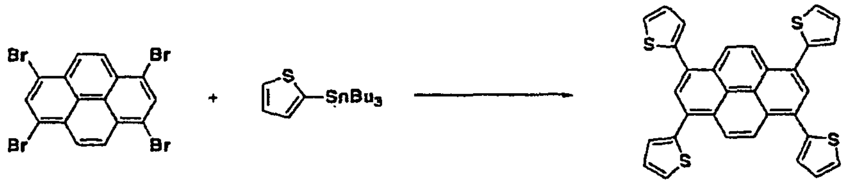

<1>

In accordance with the above-mentioned reaction formula <1>, tetrakis(2-thienyl)pyrene ((3-1) in Fig. 1(a)) was produced. Specifically, a 300 ml four-necked flask having a reflux condenser tube and a three-way cock connected to a nitrogen line was charged with 10.3 g of 2-thienyltributyltin (reagent made by Tokyo Kasei Kogyo Co., Ltd.), 2.0 g of 1,3,6,8-tetrabromopyrene described above, and 200 ml of dehydrated toluene (reagent made by Kanto Chemical Co., Inc.). The reactor was purged with nitrogen, and then the reaction solution was further bubbled with nitrogen so as to degas the solution. 0.2 g of tetrakistriphenylphosphine palladium (0) (reagent made by Tokyo Kasei Kogyo Co., Ltd.) was added thereto, and then the mixture was refluxed in an oil bath at 110°C for 6 hours, and then left at rest overnight in the atmosphere of nitrogen.

The reaction solution was filtered through celite, and the remaining solid was washed off with chloroform. The filtrate was successively washed with a 10% aqueous solution of potassium fluoride, pure water, and saturated salt water. Sodium sulfate was used to dehydrate the solution, and then the resultant was concentrated with an evaporator to yield 1 g of yellow microcrystals.

The collected crystals were purified by GPC to yield 0.4 g of a single component. From mass spectrometry based on ionization by DEI, this was identified as 1,3,6,8-tetrakis(2-thienyl)pyrene (yield: 18%). Data about the mass spectrometry (MS) are as follows:

·MS:m/z=40,162,206,248,265,401,451,485,530

(Production Example 2) Production of tetrakis(4-biphenyl)pyrene (chemical formula (3-9))

[Chemical formula 5]

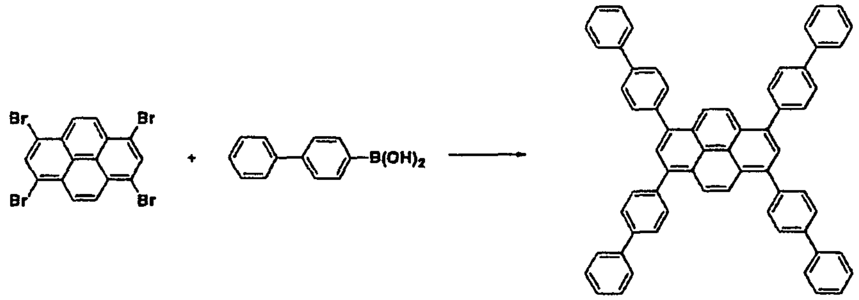

<2>

In accordance with the above-mentioned reaction formula <2>, tetrakis(4-biphenyl)pyrene ((3-9) in Fig. 2(b)) was produced. Specifically, a 500 ml four-necked flask having a reflux condenser tube, a three-way cock connected to

a nitrogen line, and a thermometer were charged with 2.3 g of 4-biphenylboric acid (reagent made by Aldrich Co.), 1.0 g of 1,3,6,8-tetrabromopyrene described above, 6.4 g of cesium carbonate (reagent made by Kishida Chemical Co., Ltd.), 150 ml of toluene, 60 ml of ethanol (reagent made by Junsei Chemical Co., Ltd.), and 30 ml of pure water. The pressure in the reactor was reduced to degas the reactor 5 times. Then, nitrogen was caused to flow into the reaction solution. Next, 0.2 g of tetrakistriphenylphosphine palladium (0), was added thereto and the resultant was refluxed in an oil bath at 80°C for 9 hours, and left at rest overnight in the atmosphere of nitrogen.

To the reaction mixture, 100 ml of chloroform and 100 ml of pure water were added, and a solid insoluble in the two solvents was collected by suction filtration. (The filtrate was separated to phases, and the organic phase thereof was washed twice with 100 ml of pure water.)

The collected solid was purified by column chromatography (silica gel, chloroform) so as to remove palladium mixed therewith. Thereafter, the resultant was recrystallized from chloroform to collect 745 mg of yellow needle-like crystals. From mass spectrometry based on ionization by MALDI, this was identified as 1,3,6,8-tetrakis(4-biphenyl)pyrene (yield: 47%). Data about the mass spectrometry (MS) are as follows:

·MS:m/z=658,810

(Production Example 3) Production of tetrakis(3-biphenyl)pyrene (chemical formula (3-8))

[Chemical formula 6]

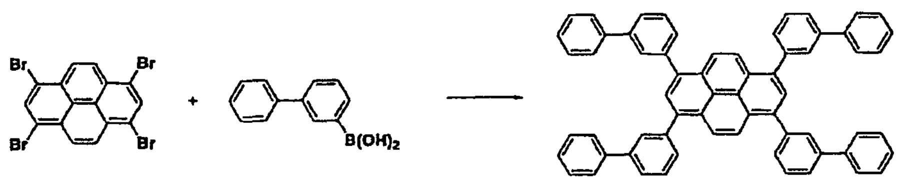

<3>

In accordance with the above-mentioned reaction formula <3>, tetrakis(3-biphenyl)pyrene ((3-8) in Fig. 2(b)) was produced. Specifically, a 500 ml four-necked flask equipped with a reflux condenser tube, a three-way cock, and a thermometer were charged with 4.7 g of 3-biphenylboric acid (reagent made by Aldrich Co.), 2.5 g of 1,3,6,8-tetrabromopyrene described above, 250 ml of toluene, and 80 ml of ethanol. The reactor was degassed by reducing the pressure therein. Then, bubbling with nitrogen was carried out. An aqueous solution obtained by dissolving 5.1 g of sodium carbonate (reagent made by Kanto Chemical Co., Inc.) in 25 ml of pure water and bubbling with nitrogen was added thereto. The resultant mixture was further bubbled with nitrogen. Next, 0.4 g of the abovementioned tetrakistriphenylphosphine palladium (0) was added thereto, and the resultant was refluxed in an oil bath at 80°C for 8 hours. The mixture was cooled, and then 200 ml of chloroform and 200 ml of pure water were added thereto so as to separate the solution into phases. The collected organic phase was concentrated with an evaporator. The residue was dissolved into hot chloroform, and the resultant was heated and filtered to remove inorganic salts. The filtrate was concentrated to collect a solid. GPC was used to purify 0.5 g out of 1 g of the collected solid, so as to collect 342 mg of a single component. From mass spectrometry based on ionization by DEI, this component was identified as 1,3,6,8-tetrakis(3-biphenyl)pyrene (yield: 9%). Data about the mass spectrometry (MS) are as follows: ·MS: m/z=154, 289, 405, 503, 578, 655, 656, 732, 810

(Production process 4) Production of 1,3,6,8-tetrakis(3-.tolyl)pyrene (chemical formula (3-1))

[Chemical formula 7]

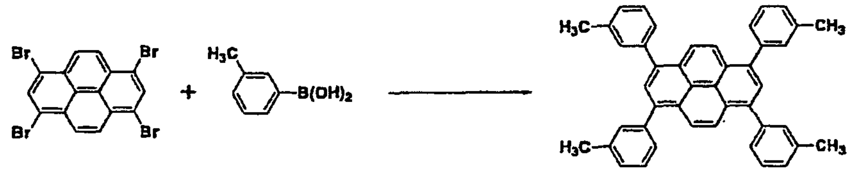

<4>

To 15 g of 3-tolylboronic acid (reagent made by Tokyo Kasei Kogyo Co., Ltd.), 9.2 g of 1,3,6,8-tetrabromopyrene, and 6.4 g of cesium carbonate (reagent made by Kishida Chemical Co., Ltd.), 400 ml of toluene (reagent made by Junsei Chemical Co., Ltd.), 50 ml of ethanol (reagent made by Junsei Chemical Co., Ltd.) and 50 ml of pure water were added, and then the mixture was purged with nitrogen. Thereafter, 2 g of tetrakistriphenylphosphine palladium (0) (reagent made by Tokyo Kasei Kogyo Co., Ltd.) was added thereto. The resultant was heated and refluxed for 7 hours.
The reaction solution was concentrated under reduced pressure, and 100 ml of water was added thereto. The resultant was extracted with dichloromethane several times, and sodium sulfate was added to the extracted liquid so as to dehydrate the liquid. The liquid was filtered and concentrated, and then the resultant residue was recrystallized from toluene to yield 3.7 g of a yellow solid. From FAB mass spectrometry thereof, a result of m/z = 563 was obtained. It was understood from this fact that this component was 1,3,6,8-tetrakis(3-tolyl)pyrene.

(Production process 5) Production of tetrakis(4-fluorophenyl)pyrene (chemical formula (3-13))

[Chemical formula 8]

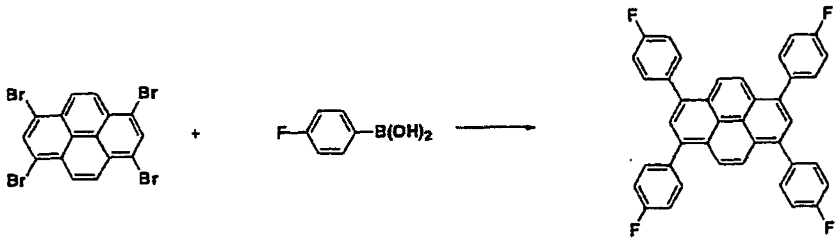

<5>

To 8.4 g of 4-fluorophenylboronic acid (reagent made by Aldrich Co.), 5.2 g of 1,3,6,8-tetrabromopyrene, and 20 g of cesium carbonate (reagent made by Kishida Chemical Co., Ltd.), 200 ml of toluene (reagent made by Junsei Chemical Co., Ltd.), 25 ml of ethanol (reagent made by Junsei Chemical Co., Ltd.) and 25 ml of pure water were added, and then the system was purged with nitrogen. Thereafter, 1 g of tetrakistriphenylphosphine palladium (0) (reagent made by Tokyo Kasei Kogyo Co., Ltd.) was added thereto. The resultant was heated and refluxed for 9 hours.
The reaction solution was filtered, and then the resultant residue was washed with methanol and recrystallized from toluene to yield 4.3 g of a yellow solid. From FAB mass spectrometry thereof, peaks of 578($M^+$) and 540 were obtained. It was understood from this fact that this component was 1,3,6,8-tetrakis(4-fluorophenyl)pyrene.

(Production process 6) Production of tetrakis(3,5-difluorophenyl) pyrene (chemical formula (3-16))

[Chemical formula 9]

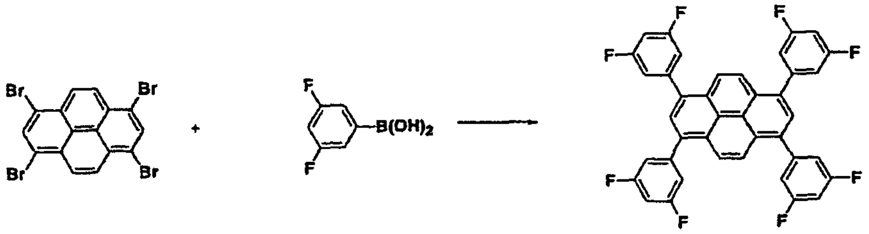

<6>

To 9.5 g of 3,5-fluorophenylboronic acid (reagent made by Aldrich Co.), 5.2 g of 1,3,6,8-tetrabromopyrene, and 20 g of cesium carbonate (reagent made by Kishida Chemical Co., Ltd.), 200 ml of toluene (reagent made by Junsei Chemical Co., Ltd.), 25 ml of ethanol (reagent made by Junsei Chemical Co., Ltd.) and 25 ml of pure water were added, and then the system was purged with nitrogen. Thereafter, 1 g of tetrakistriphenylphosphine palladium (0) (reagent made by Tokyo Kasei Kogyo Co., Ltd.) was added thereto. The resultant was heated and refluxed for 9 hours.
The reaction solution was filtered, and then the resultant residue was washed with methanol and recrystallized from toluene to yield 4.2 g of a yellow solid. From FAB mass spectrometry thereof, 650($M^+$) was obtained. It was understood from this fact that this component was 1,3,6,8-tetrakis(4-fluorophenyl)pyrene.

(Production process 7) Production of 1,3,6,8-tetrakis(4-fluorophenyl) pyrene (chemical formula (4-2))

[Chemical formula 10]

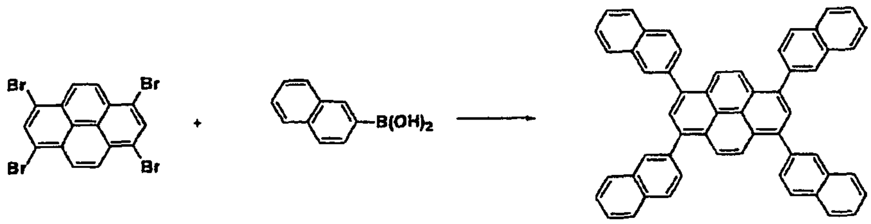

<7>

To 10.3 g of 2-naphthylboronic acid (reagent made by Tokyo Kasei Kogyo Co., Ltd.), 5.2 g of 1,3,6,8-tetrabromopyrene, and 20 g of cesium carbonate (reagent made by Kishida Chemical Co., Ltd.), 200 ml of toluene (reagent made by Junsei Chemical Co., Ltd.), 25 ml of ethanol (reagent made by Junsei Chemical Co., Ltd.) and 25 ml of pure water were added, and then the system was purged with nitrogen. Thereafter, 1 g of tetrakistriphenylphosphine palladium (0) (reagent made by Tokyo Kasei Kogyo Co., Ltd.) was added thereto. The resultant was heated and refluxed for 9 hours.
The reaction solution was filtrated, and then the resultant residue was washed with hot water and recrystallized from toluene to yield 5.7 g of a yellow solid. It was understood from an FAB mass spectrometry described below that this was 1,3,6,8-tetrakis(4-fluorophenyl)pyrene.
·MS:m/z=55,180,254,523,549,706

(Production Example 8) Production of 1,3,6,8-tetrakis(trans-styryl)-pyrene

[Chemical formula 11]

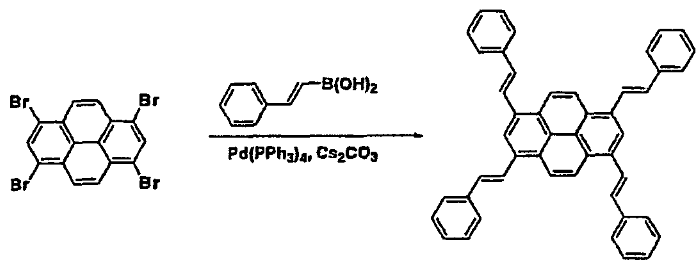


<8>

A 500 ml four-necked flask having a reflux condenser tube, a three-way cock and a thermometer were charged with 15 g of trans-styryl boric acid (reagent made by Tokyo Kasei Kogyo Co., Ltd.), 10 g of 1,3,6,8-tatrabromopyrene, 33 g of cesium carbonate (reagent made by Kishida Chemical Co., Ltd.), 400 ml of toluene (reagent made by Junsei Chemical Co., Ltd.), 50 ml of ethanol (reagent made by Junsei Chemical Co., Ltd.) and 50 ml of pure water, and then the system was purged with nitrogen. Thereafter, 2 g of tetrakistriphenylphosphine palladium (0) (reagent made by Tokyo Kasei Kogyo Co., Ltd.) was added thereto. The resultant was heated and refluxed in an oil bath at 80°C for 9 hours.
To the reaction mixture, 100 ml of $CHCl_3$ and 100 ml of pure water were added, and the resultant solution was filtrated to yield 6.6 g of a yellow solid. From FAB mass spectrometry of the resultant solid, a result of m/z = 611 was obtained. It was understood from this matter that this component was 1,3,6,8-tetrakis(trans-styryl)pyrene.

(Production Example 9) Production of 1,3,6,8-tetrakis(4-tolyl)pyrene

[Chemical formula 12]

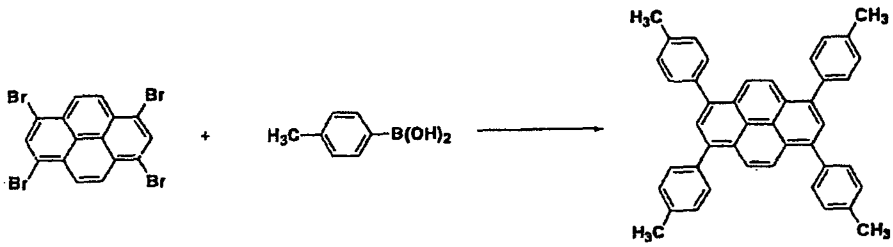

<9>

To 8.0 g of 4-tolylboronic acid (reagent made by Tokyo Kasei Kogyo Co., Ltd.), 5.0 g of 1,3,6,8-tetrabromopyrene, and 31 g of cesium carbonate (reagent made by Kishida Chemical Co., Ltd.), 200 ml of toluene (reagent made by Junsei Chemical Co., Ltd.), 100 ml of ethanol (reagent made by Junsei Chemical Co., Ltd.) and 40 ml of pure water were added, and then the system was purged with nitrogen. Thereafter, 0.6 g of tetrakistriphenylphosphine palladium (0) (reagent made by Tokyo Kasei Kogyo Co., Ltd.) was added thereto. The resultant was heated and refluxed for 7 hours.
The reaction solution was concentrated under reduced pressure, and 100 ml of water was added thereto. The resultant was extracted with chloroform, and sodium sulfate was added to the extracted liquid so as to dehydrate the liquid. The liquid was filtered and concentrated, and then the resultant residue was purified by GPC, so as to yield 0.8 g of a yellow solid. From FAB mass spectrometry thereof, it was understood that this component was 1,3,6,8-tetrakis(4-tolyl)pyrene.
·MS:m/z=69,109,145,180,207,256,281,307,424,456,472,523,561,562

(Production Example 10) Production of 1,3,6,8-tetrakis(3,5-bis (trifluoromethyl)phenyl)pyrene (the following chemical formula <10>)

[Chemical formula 13]

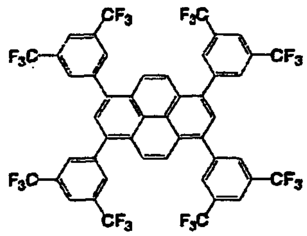

<10>

A 200 ml three-necked flask having a reflux condenser tube, a three-way cock and a thermometer were charged with 5.2 g of 3,5-bis(trifluoromethyl)phenylboric acid (reagent made by Aldrich Co.), 1.5 g of 1,3,6,8-tatrabromopyrene, 4.3 g of sodium carbonate (reagent made by Kanto Chemical Co., Inc.), 50 ml of toluene (reagent made by Junsei Chemical Co., Ltd.), 15 ml of ethanol (reagent made by Junsei Chemical Co., Ltd.) and 10 ml of pure water. The pressure in the reactor was reduced to degas the reactor 5 times. Furthermore, nitrogen was caused to pass into the reactor. 0.3 g of tetrakistriphenylphosphine palladium (0) (reagent made by Tokyo Kasei Kogyo Co., Ltd.) was added thereto. The resultant was refluxed in an oil bath at 80°C for 12 hours, and left at rest overnight in the atmosphere of nitrogen.
To the reaction mixture, 100 ml of $CHCl_3$ and 150 ml of pure water were added to separate this solution into phases. The water phase was extracted twice with 100 ml of $CHCl_3$. Anhydrous magnesium sulfate was used to dry the organic phase, and then the phase was concentrated. The residue was washed with acetonitrile, and the resultant precipitation was collected. This was further purified by GPC. From mass spectrometry based on DEI ionization, a result of m/Z = 1050 was obtained so that this component was identified as 1,3,6,8-tetrakis(3,5-bis(trifluoromethyl) phenyl)pyrene (yielded amount: 0.59 g, yield: 19%).
·$^1$H NMR(400MHz, $CDCl_3$)δ8.12(br, 8H), 8.09(s, 4H), 8.05(br, 4H), 8.02(s, 2H)
·Mass(DEI)Obs.m/Z=1050($M^+$), Calc. for C48H18F24

(Production Example 11) Production of 1,3,6,8-tetrakis (4-trifluoromethylphenyl)pyrene (the following chemical formula <11>)

[Chemical formula 14]

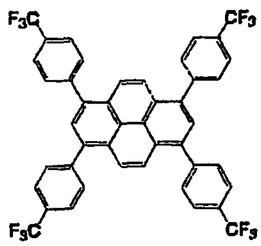

<11>

A 200 ml three-necked flask having a reflux condenser tube, a three-way cock and a thermometer were charged with 3.0 g of p-trifluoromethylphenylboric acid (reagent made by Aldrich Co.), 1.4 g of 1,3,6,8-tatrabromopyrene, 3.4 g of sodium carbonate (reagent made by Kanto Chemical Co., Inc.), 50 ml of toluene (reagent made by Junsei Chemical Co., Ltd.), 15 ml of ethanol (reagent made by Junsei Chemical Co., Ltd.) and 10 ml of pure water. The pressure in the

reactor was reduced to degas the reactor 5 times. Furthermore, nitrogen was caused to pass into the reactor. Thereto, 0.3 g of tetrakistriphenylphosphine palladium (0) (reagent made by Tokyo Kasei Kogyo Co., Ltd.) was added. The resultant was refluxed in an oil bath at 80°C for 12 hours, and left at rest overnight in the atmosphere of nitrogen.
To the reaction mixture, 50 ml of pure water was added to separate this solution into phases. Furthermore, the water phase was extracted with 50 ml of toluene two times. Anhydrous magnesium sulfate was used to dry the combined organic phase, and then the resultant phase was concentrated. The resultant solid was washed with $CHCl_3$, and the collected solid was recrystallized from toluene. (Yielded amount: 0.55 g, yield: 27%).
·$^1$H NMR(400MHz, CDC13)δ8.13(s,4H), 7.99(s, 2H), 7.83-7.77(m, 16H)

(Examples 1 to 7)

Next, a light emitting transistor element illustrated in Figs. 5 and 6 was manufactured.
Source electrode 2 and drain electrode 3: Electrodes (Au, thickness: 40 nm) each having a comb tooth-shaped region comprising 20 comb teeth were formed. As shown in Fig. 7, the individual comb tooth-shaped regions were formed on an insulating film 5 in such a manner that the regions were alternately arranged. At this time, a layer (1 nm) made of chromium was formed between the insulating film 5 and each of the two electrodes. The channel region (between the individual comb tooth shaped regions) at this time was designed to have a width of 25 μm and a length of 4 mm.
Insulating film 5: A silicon oxide film 300 nm in thickness was formed by vapor deposition.
Light emitting layer 1: The pyrene based compounds (2-1), (3-9), (3-8), (3-1), (3-16), (4-2) and (3-6), obtained by the above-mentioned production processes, were each independently deposited to cover the periphery of the insulating film, the source electrode 2 and the drain electrode 3, thereby providing the light emitting layer 1.
For each of the elements, the HOMO and LUMO energy levels, the fluorescence absorption wavelength, the PL luminous efficiency, the EL luminous efficiency, the luminous brightness, and the carrier mobility thereof were measured. The results are shown in Table 1.
The carrier mobility, the EL luminous efficiency, and the PL luminous efficiency were measured/calculated as follows:

(Carrier mobility μ ($cm^2/V_s$))

A relational expression between the drain voltage ($V_d$) and the drain current of an organic semiconductor is represented by the following expression (1), and it increases linearly (linear area),
**[0093]**

[Expression 1]

$$I_d = \frac{W}{L}\mu C_i\left[(V_g - V_T)V_d - \frac{1}{2}V_d^2\right] \quad (1)$$

When $V_d$ increases, $I_d$ is saturated by the pinch-off of the channel, so that $I_d$ becomes a constant value (saturated area) and is represented by the following expression (2).
[Expression 2]

$$I_d = \frac{W}{2L}\mu_{sat} C_i (V_g - V_T)^2 \quad (2)$$

In the expressions (1) and (2), each of the symbols is as follows:

L: channel length [cm],
W: channel width [cm],
$C_i$: electrostatic capacity [F/cm$^2$] of the gate insulating film per unit area,
$\mu_{sat}$: mobility [cm$^2$/Vs] in the saturate area,
$I_d$: drain current [A],
$V_d$ : drain voltage [V],
$V_g$: gate voltage [V], and
$V_T$: gate threshold voltage [V], which represents the following point: in a graph obtained by plotting the 1/2 power of the drain current ($V_{dsat}{}^{1/2}$) versus the gate voltage ($V_g$) under a condition that the drain voltage ($V_d$) in the saturated area is constant, a point at which the asymptotic line therein intersects the transverse axis.

From the relationship between $I_d{}^{1/2}$ and $V_g$ in this saturated area, the mobility ($\mu$) in the organic semiconductor can be obtained.

In the present invention, under conditions that the pressure is set into the range of vacuum to $5 \times 10^{-3}$ and the temperature is set to room temperature, a Semiconductor Parameter Analyzer (HP4155C, Agilent) was used, and this was operated to set the drain voltage from 10 V to 100 V at intervals of -1V, and set to the gate voltage from 0 to -100 V at intervals of -20 V. The mobility was then calculated by use of the expression (2).

(EL luminous efficiency)

About the EL luminous efficiency $\eta_{ext}$, the above-mentioned transistor elements were each used, and operations were made to set the drain voltage from 10 V to -100 V at intervals of -1 V, and set to the gate voltage from 0 to -100 V at intervals of -20 V. Light emitted from the element was measured with a photon counter (4155C, Semiconductor Parameter Analyzer, manufactured by Newport Co.). The following expression (3) was used to convert the number of photons [CPS] obtained therein to the light fluxes [lw], and subsequently the following expression (4) was used to calculate out the EL luminous efficiency $\eta_{ext}$.

[Expression 3]

$$X_{PC}[h\nu] = \frac{5.71 \times 10^{-11}\ (N_{PC}[CPS] - \text{base})\ \frac{4}{3}\pi r^3 / \frac{h}{3}\pi r^2}{1.04 \times 10^6} \qquad (3)$$

[Expression 4]

$$\eta_{ext} = (100 \times 1239.7/\lambda \times N_{PC} \times X_{PC}) / I_d \qquad (4)$$

In the expressions (3) and (4), each of the symbols is as follows:

$N_{PC}$: number of photons [CPS] measured with the photon counter (PC),
$X_{PC}$: numerical value obtained by converting the number of the photons to light fluxes [1w],
r: diameter [cm] of the cone or circle, and
h: distance [cm] between the photon counter and the sample.

(PL luminous efficiency)

The PL luminous efficiency was calculated by vapor deposition of each of the materials obtained in the present invention into a thickness of 70 nm onto a quartz substrate in the atmosphere of nitrogen to form a mono-layered film, using an integrating sphere (IS-060, Labsphere Co.) to radiate a He-Cd laser (IK5651R-G, Kimmon Electric Co.) having

a wavelength of 325 nm as an exciting ray, and then measuring a light emitting Multi-channel photodiode (PMA-11, Hamamatsu Photonics Co.) from the sample.

(Comparative Example 1)

Measurements were made in the same way as in Example 1 except that tetraphenylpyrene (reagent made by Aldrich Co.) was used as a pyrene based compound. The results are shown in Table 1.
Table 1

| | Examples | | | | | | | Comparative Example |
|---|---|---|---|---|---|---|---|---|
| | 1 | 2 | 3 | 4 | 5 | 6 | 7 | 1 |
| Compound | (2-1) | (3-9) | (3-8) | (3-1) | (3-16) | (4-2) | (3-6) | TPPy |
| HOMO/LUMO energy levels (eV) | 5.4/2.8 | 5.6/2.8 | 5.6/2.6 | 5.4/2.4 | 6.0/3.3 | 5.4/2.7 | 5.1/2.8 | 5.6/2.7 |
| Fluorescence absorption wavelength (nm) | 534 | 533 | 456 | 470 | 487 | 499 | 592 | 505 |
| PL luminous efficiency (%) | 21 | 54 | 49 | 73 | 31 | 53 | <1 | 34 |
| EL luminous efficiency (%) | $2\times10^{-3}$ | $8\times10^{-2}$ | $2\times10^{-2}$ | $3.9\times10^{-2}$ | - | $8.6\times10^{-3}$ | $4.9\times 10^{-4}$ | $5,5\times10^{-5}$ |
| Luminous brightness (CPS) | $7.6\times10^{4}$ | $3.5\times10^{6}$ | $4.3\times10^{5}$ | $1.2\times10^{6}$ | - | $1.7\times10^{6}$ | $7.1\times10^{4}$ | $1.2\times10^{5}$ |
| Carrier mobility ($cm^2/V\cdot S$) | $3.3\times10^{-5}$ | $1.7\times10^{-4}$ | $6.7\times10^{-5}$ | $4.7\times10^{-5}$ | - | $2.3\times10^{-4}$ | $2.0\times10^{-4}$ | $9.0\times10^{-2}$ |

## Claims

1. A pyrene based compound represented by the following chemical formula (1):

[Chemical formula 1]

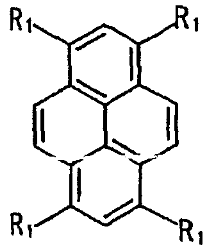

(1)

(wherein $R_1$ represents a group selected from a heteroaryl group which may have a substituent, an aryl group which may have a substituent (except a phenyl group which does not have any substituent), an alkyl group which may have a substituent and has a main chain having 1 to 20 carbon atoms, an alkenyl group which may have a substituent, an alkynyl group which may have a substituent, a silyl group which may have a substituent, and a group having a halogen atom.)

2. The pyrene based compound according to claim 1, wherein $R_1$ in the formula (1) is a group selected from benzofuryl, pyrrolyl, benzoxazolyl, pyrazinyl, thienyl, pyridyl, quinolyl, benzothiazolyl, naphthyl, anthryl, phenanthryl, vinyl, ethynyl and silyl groups each of which may have a substituent, a phenyl group which has a substituent, a carboxyl group, and a halogen atom.

3. The pyrene based compound according to claim 1 or 2, wherein each $R_1$ in the formula (1) is a group selected from carboxyl, benzofuryl, pyrrolyl, benzoxazolyl, pyrazinyl, thienyl, alkyl-substituted thienyl, bithienyl, phenyl-thienyl, benzothienyl, pyridyl, bipyridyl, phenyl-pyridyl, quinolyl, benzothiazolyl, 2-naphthyl, 2-anthryl, phenanthryl, methylphenyl, ethylphenyl, dimethylphenyl, phenyl-substituted vinyl, phenyl-substituted ethynyl, biphenyl, terphenyl, phenyl-etheno-phenyl, pyridine-phenyl, fluorine-substituted phenyl, ethyl-substituted vinyl, biphenyl-substituted vinyl and trimethylsilyl groups, and a fluorine atom.

4. The pyrene based compound according to any one of claims 1 to 3, which is used as a main component of a light emitting layer in a light emitting transistor.

5. A light emitting transistor element, comprising:

   a light emitting layer which is capable of transporting holes and electrons as carries, comprises the pyrene based compound according to claim 4 as a main component, and emits light by recombination of the holes and the electrons,
   a hole injecting electrode for injecting the holes into the light emitting layer,
   an electron injecting electrode for injecting the electrons into the light emitting layer, and
   a gate electrode for controlling the distribution of the carriers in the light emitting layer, the gate electrode being disposed opposite to the hole injecting electrode and the electron injecting electrode.

6. The light emitting transistor element according to claim 5, wherein the hole injecting electrode and the electron injecting electrode each have a comb tooth-shaped region comprising a plurality of comb teeth, and the comb teeth of the comb tooth-shaped region of the hole injecting electrode and the comb teeth of the comb tooth-shaped region of the electron injecting electrode are alternately arranged at predetermined intervals.

7. A display device, wherein a plurality of the light emitting transistor elements according to claim 5 or 6 are arranged on a substrate.

# Fig.1(a)

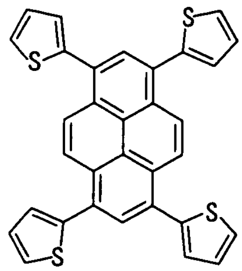

(2-1)

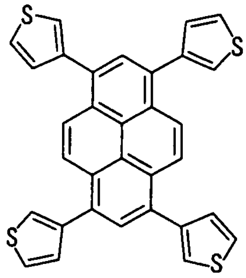

(2-2)

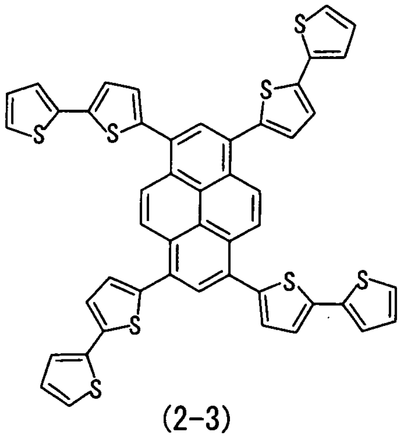

(2-3)

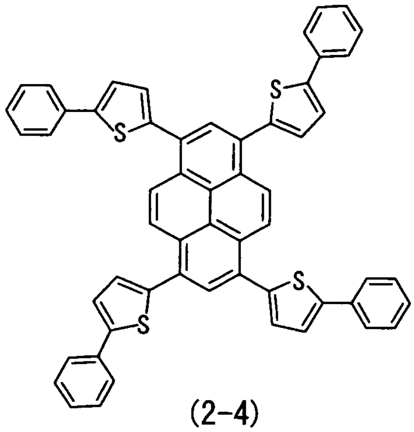

(2-4)

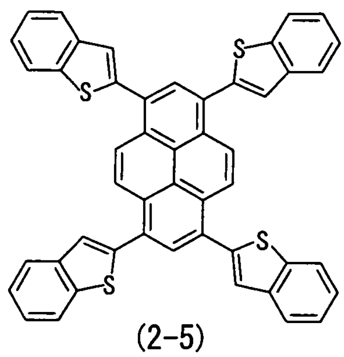

(2-5)

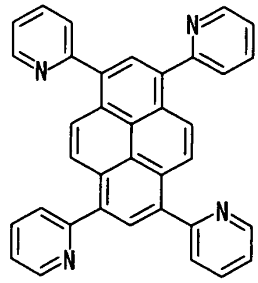

(2-6)

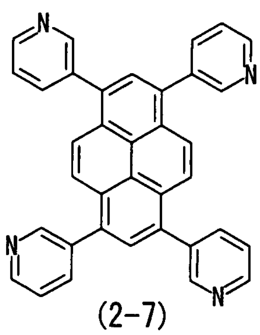

(2-7)

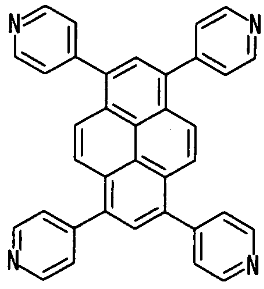

(2-8)

# Fig. 1(b)

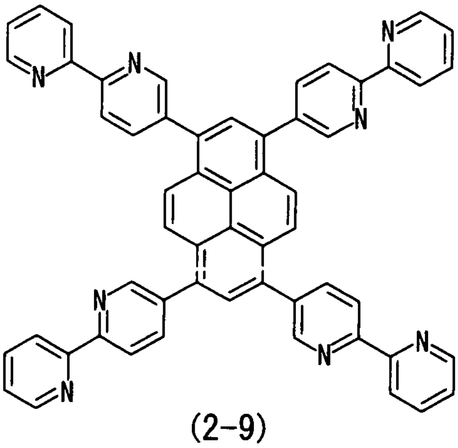

(2-9)

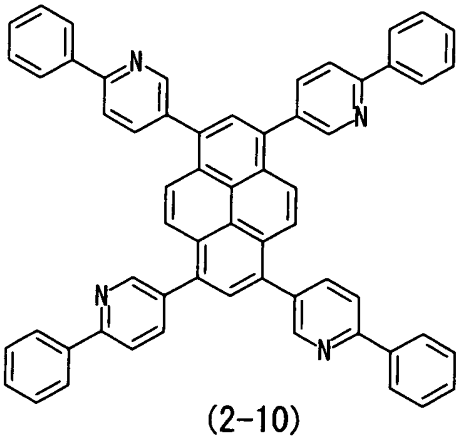

(2-10)

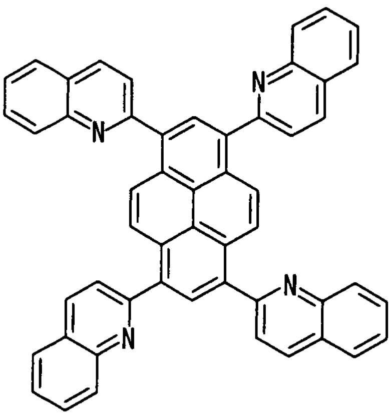

(2-11)

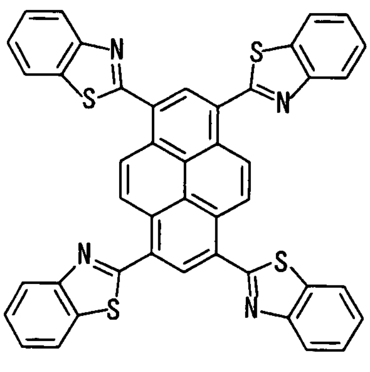

(2-12)

# Fig. 1(c)

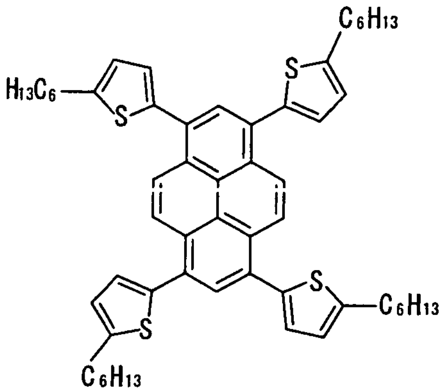

(2-13)

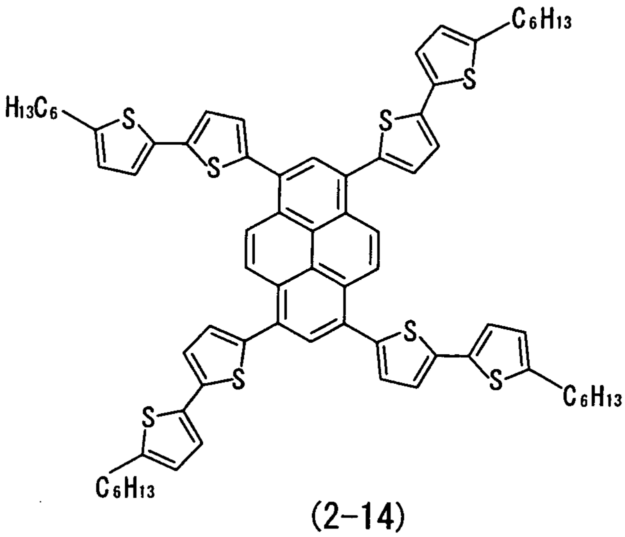

(2-14)

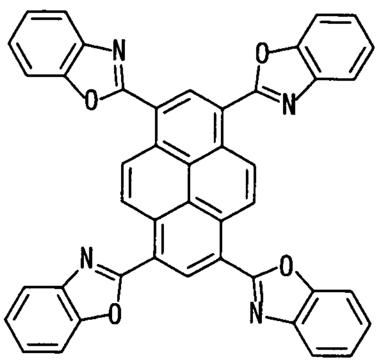

(2-15)

# Fig.2(a)

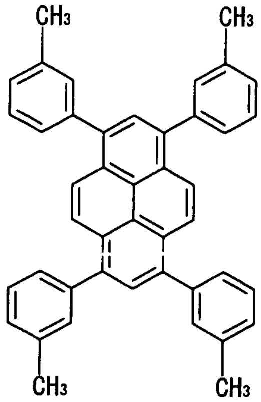

(3-1)

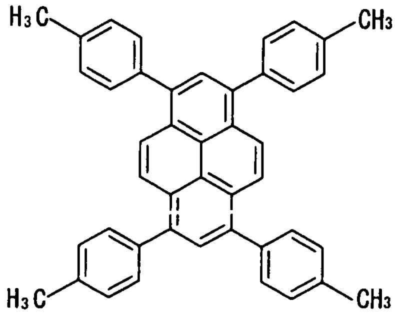

(3-2)

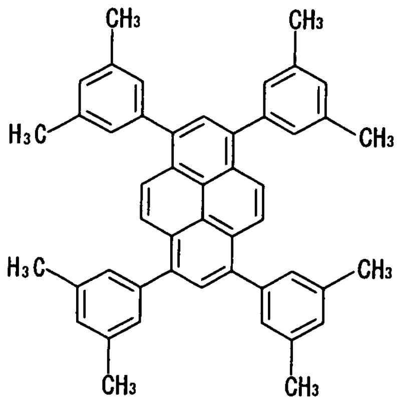

(3-3)

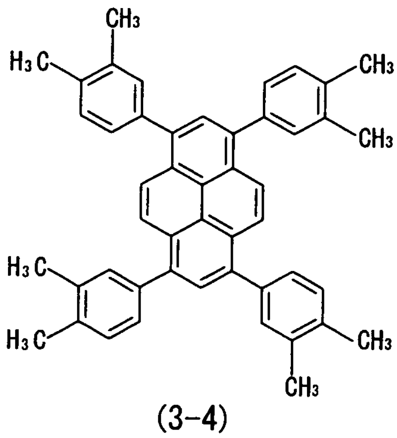

(3-4)

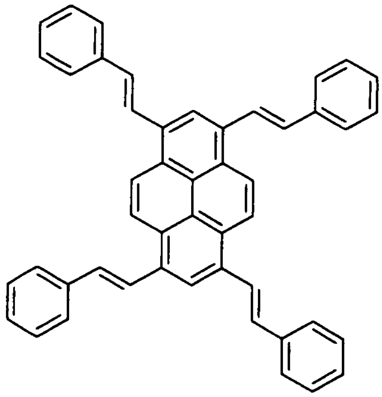

(3-5)

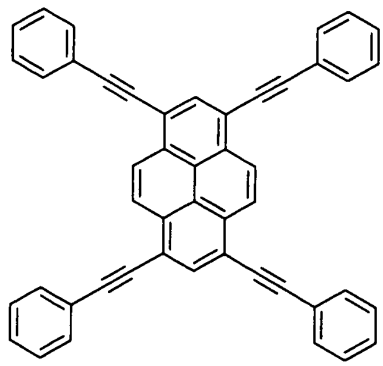

(3-6)

# Fig.2(b)

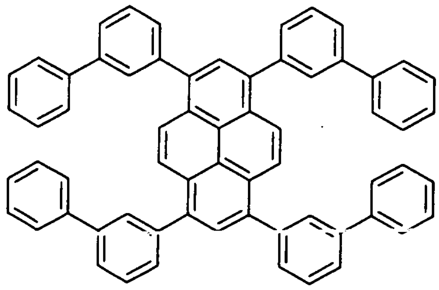

(3-7)

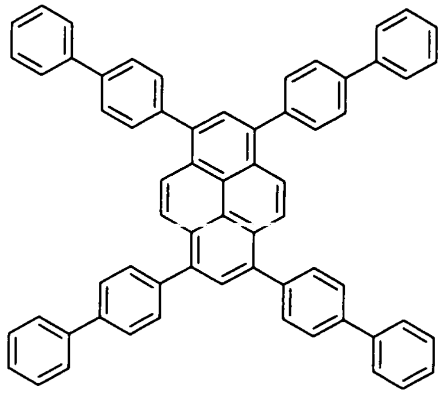

(3-8)

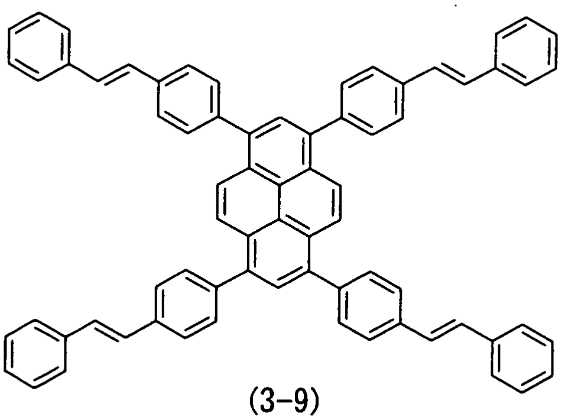

(3-9)

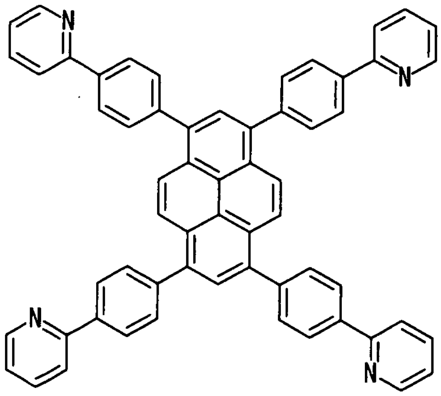

(3-10)

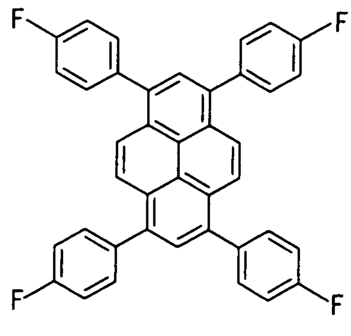

(3-11)

# Fig.2(c)

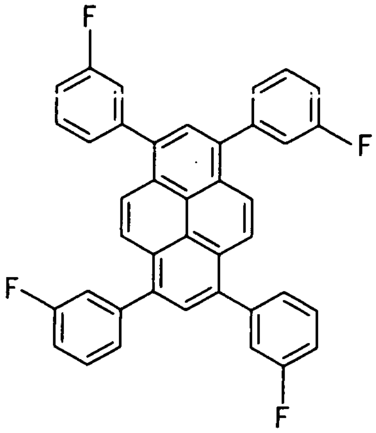

(3-12)

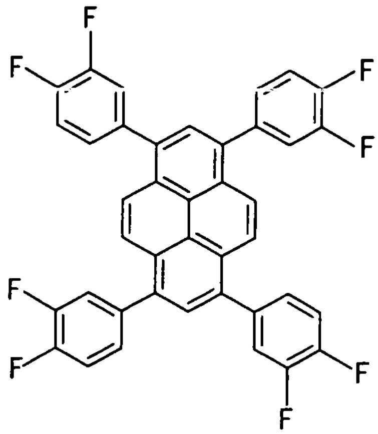

(3-13)

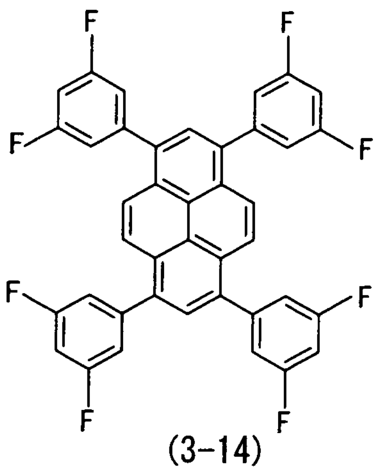

(3-14)

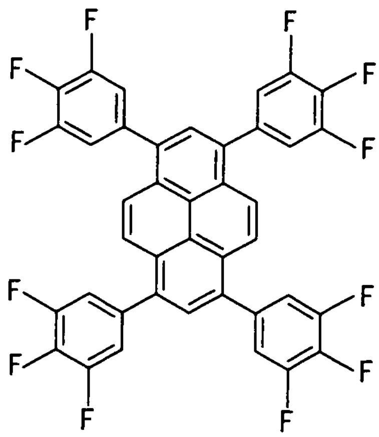

(3-15)

Fig.2(d)

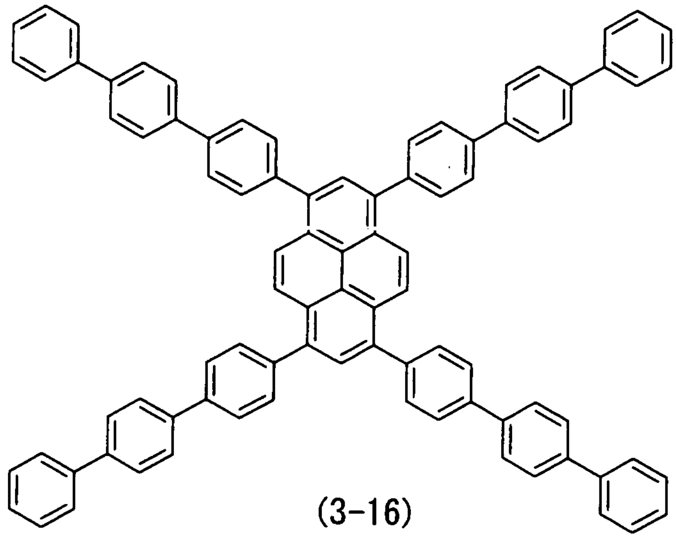

(3-16)

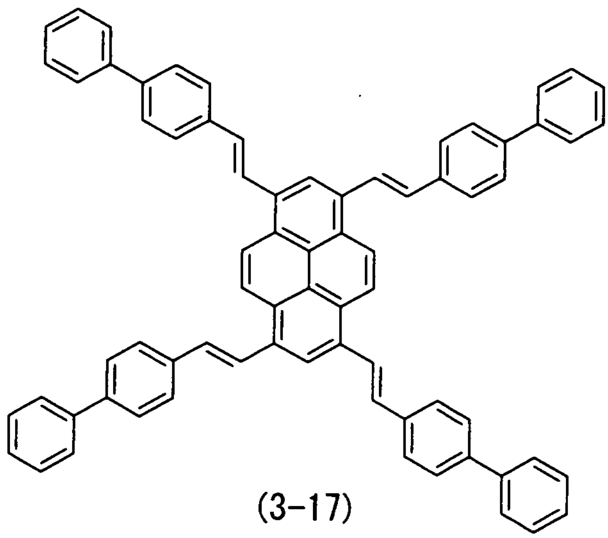

(3-17)

Fig.3(a)

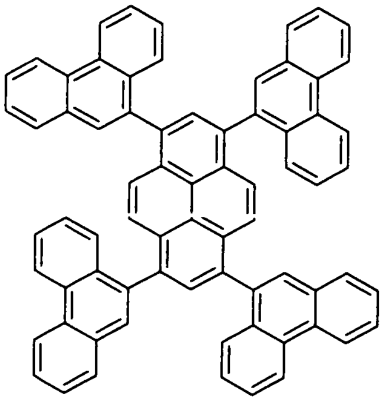

(4-1)

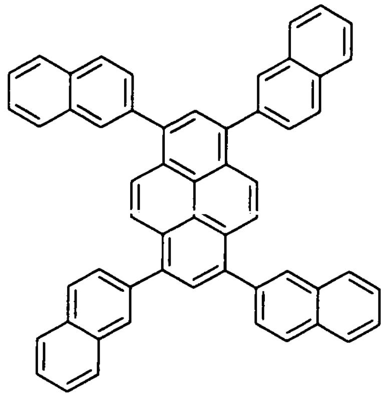

(4-2)

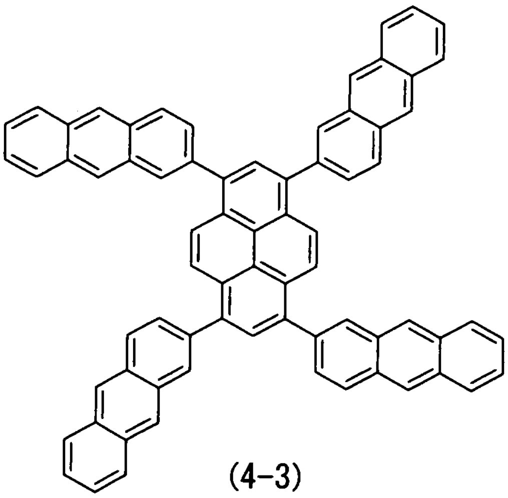

(4-3)

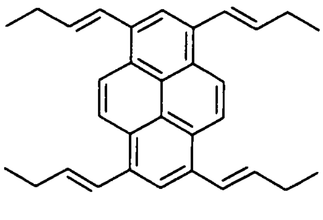

(4-4)

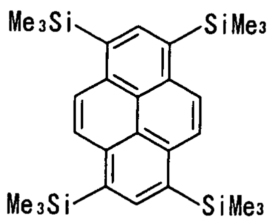

(4-5)

# Fig.3(b)

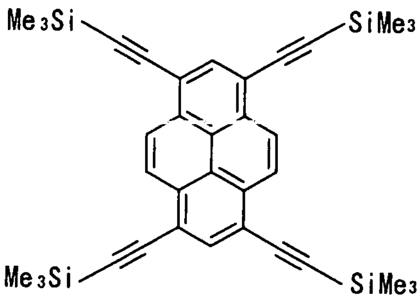

(4-6)

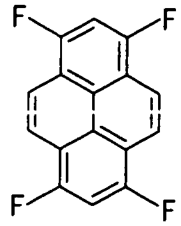

(4-7)

# Fig.4(a)

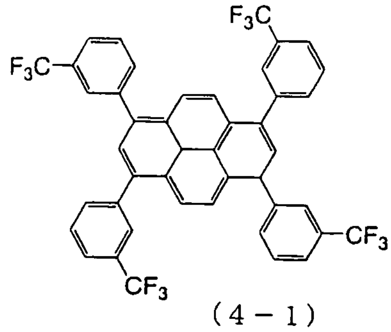

(4－1)

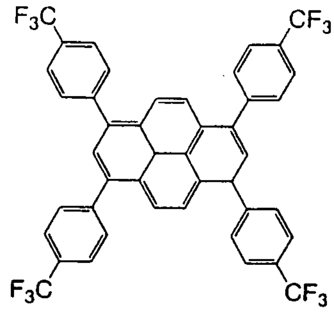

(4－2)

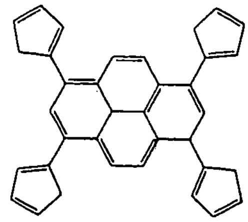

(4－3)

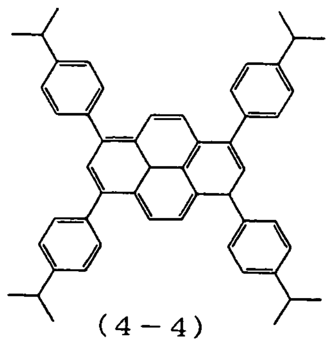

(4－4)

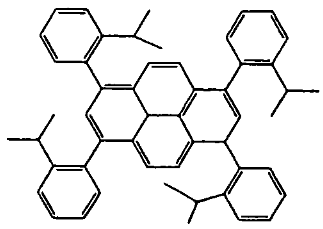

(4－5)

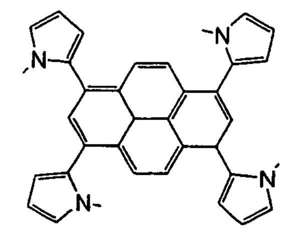

(4－6)

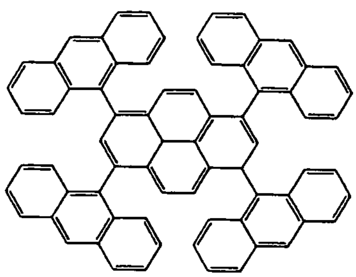

(4－7)

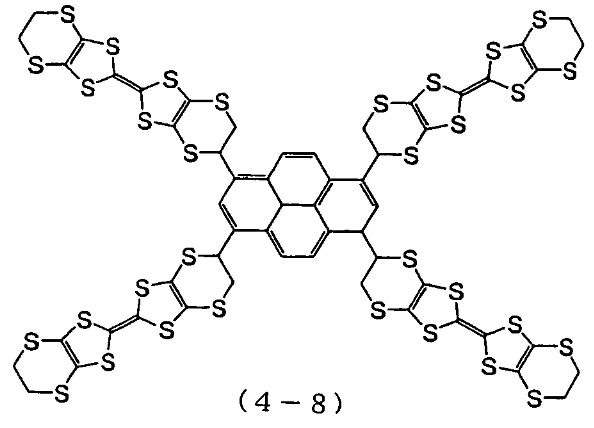

(4－8)

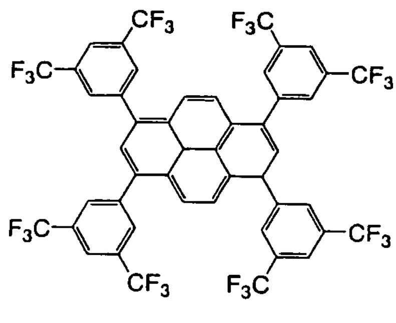

(4－9)

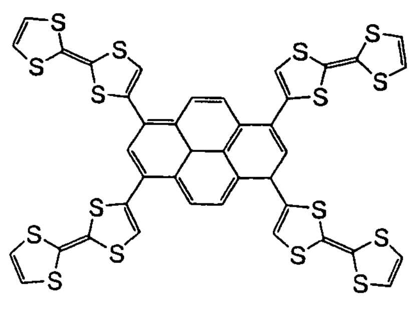

(4－10)

# Fig.4(b)

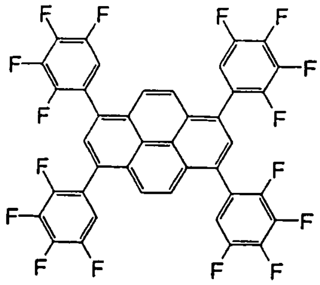

(4－1 1)

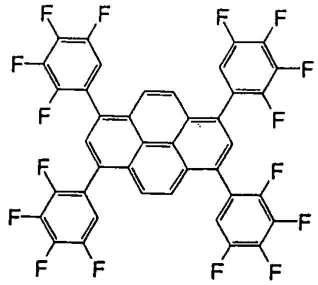

(4－1 2)

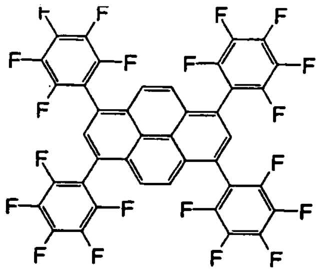

(4－1 3)

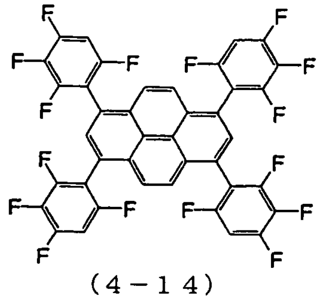

(4－1 4)

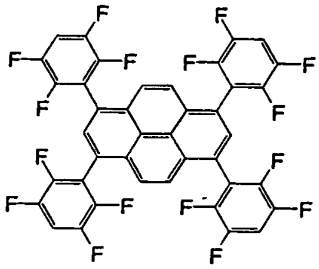

(4－1 5)

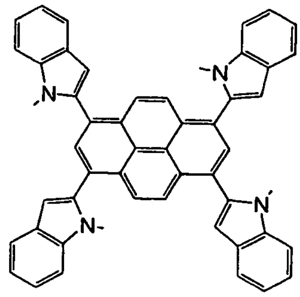

(4－1 6)

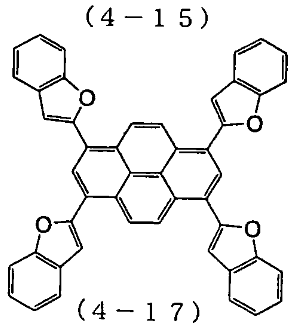

(4－1 7)

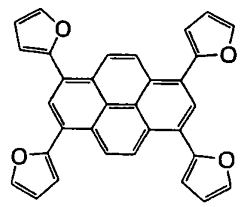

(4－1 8)

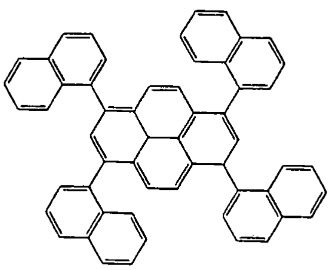

(4－1 9)

Fig.5

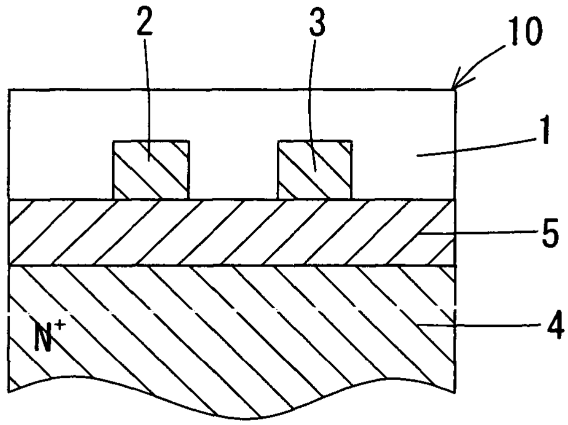


Fig.6

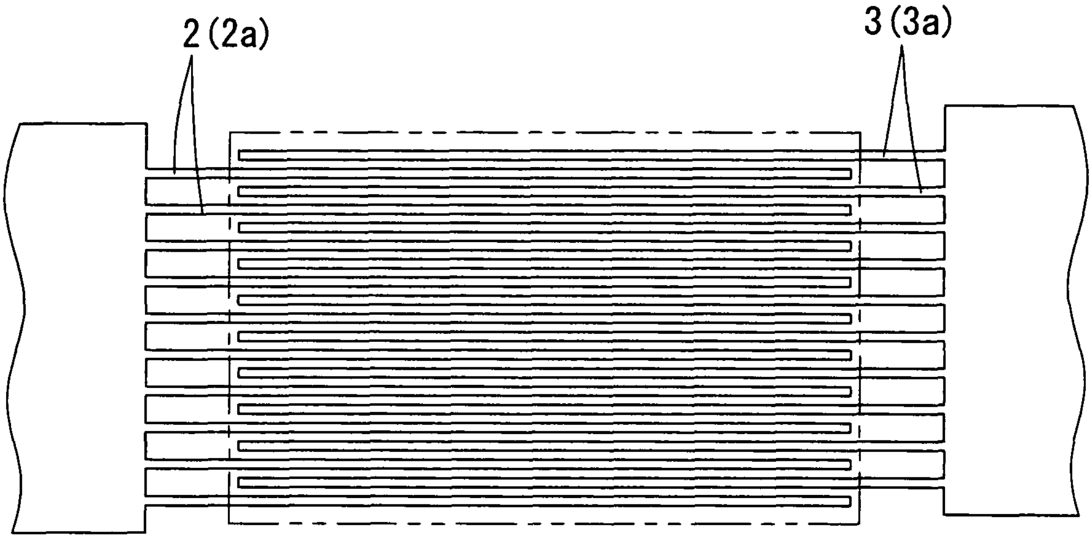

Fig.7

(a)

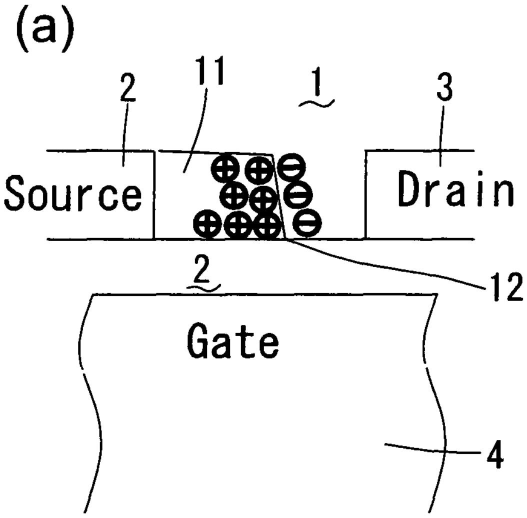


(b)

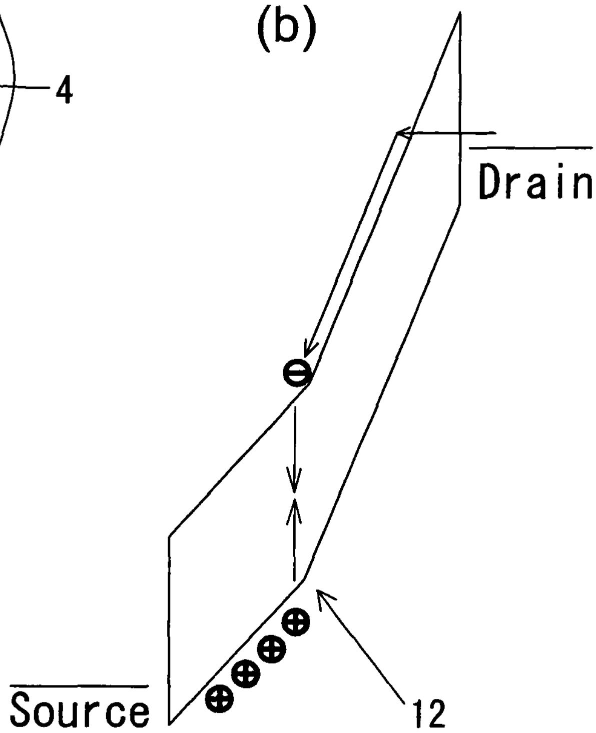


(c)

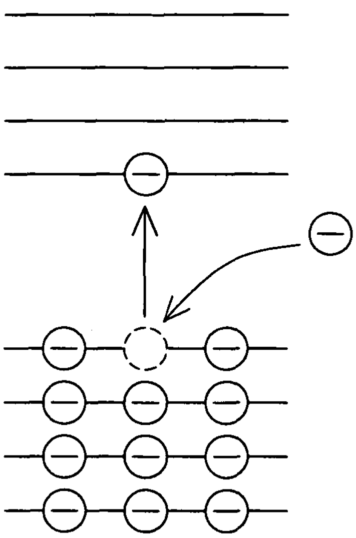

Fig.8

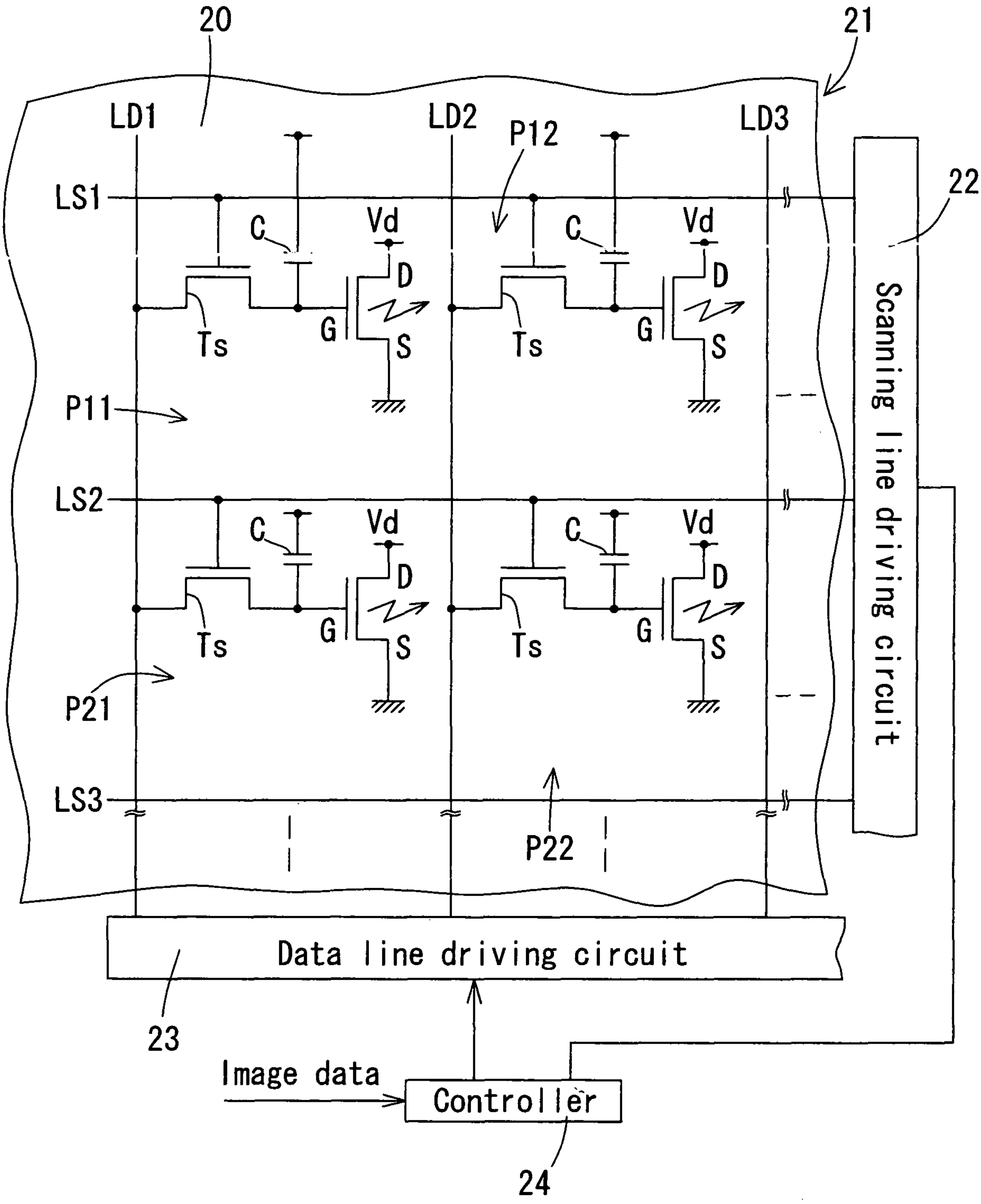
20
21
LD1
LD2
LD3
P12
LS1
C
Vd
D
G
Ts
S
P11
LS2
P21
LS3
P22
22
Scanning line driving circuit
Data line driving circuit
23
Image data
Controller
24

**INTERNATIONAL SEARCH REPORT**

International application No.
PCT/JP2005/021648

A. CLASSIFICATION OF SUBJECT MATTER
*C07C15/38*(2006.01), *C07C15/62*(2006.01), *C07C25/22*(2006.01), *C07D333/18* (2006.01), *C09K11/06*(2006.01), *H01L51/50*(2006.01), *C07C22/08*(2006.01)

According to International Patent Classification (IPC) or to both national classification and IPC

B. FIELDS SEARCHED

Minimum documentation searched (classification system followed by classification symbols)
C07C15/38, C07C15/62, C07C22/08, C07C25/22, C07D333/18, C09K11/06, H01L51/50

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2005 |
| Kokai Jitsuyo Shinan Koho | 1971-2005 | Toroku Jitsuyo Shinan Koho | 1994-2005 |

Electronic data base consulted during the international search (name of data base and, where practicable, search terms used)
CAplus(STN), REGISTRY(STN)

C. DOCUMENTS CONSIDERED TO BE RELEVANT

| Category* | Citation of document, with indication, where appropriate, of the relevant passages | Relevant to claim No. |
|---|---|---|
| X | JP 2002-63988 A (Toray Industries, Inc.), 28 February, 2002 (28.02.02), Claims 1, 2; Par. Nos. [0025], [0026] (Family: none) | 1-3 |
| X | Chemical Abstracts, Vol.136, abs.no.216806(2002) | 1-3 |
| X | WO 2004/096945 A1 (Fujitsu Ltd.), 11 November, 2004 (11.11.04), Claim 1; page 8, line 2 to page 11, 7th line from the bottom (Family: none) | 1-3 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 27 December, 2005 (27.12.05) | 17 January, 2006 (17.01.06) |
| Name and mailing address of the ISA/ Japanese Patent Office | Authorized officer |
| Facsimile No. | Telephone No. |

Form PCT/ISA/210 (second sheet) (April 2005)

**INTERNATIONAL SEARCH REPORT**

International application No.
PCT/JP2005/021648

C (Continuation). DOCUMENTS CONSIDERED TO BE RELEVANT

| Category* | Citation of document, with indication, where appropriate, of the relevant passages | Relevant to claim No. |
|---|---|---|
| X | WO 2004/096743 A1 (Fujitsu Ltd.), 11 November, 2004 (11.11.04), Claim 1; page 13, line 23 to page 17, line 22; page 28, line 1 to page 30, line 7 & US 2005/156164 A1 | 1,2 |
| X | Chemical Abstracts, Vol.73, abs.no.35116(1970) | 1 |
| X | JP 9-241629 A (Idemitsu Kosan Co., Ltd.), 16 September, 1997 (16.09.97), Claims 1, 3; Par. No. [0014] (Family: none) | 1 |
| X | Chemical Abstracts, Vol.32, abs.no.145i, 146a-i, 147a-i, 148a-i, 149a-i, 150a-i, 151a-i, 152a-e (1938) | 1,2 |
| X | JP 2003-300912 A (Fuji Photo Film Co., Ltd.), 21 October, 2003 (21.10.03), Claim 1; Par. Nos. [0092], [0094], [0101] (Family: none) | 4-7 |
| X | Chemical Abstracts, Vol.103, abs.no.87615(1985) | 1,2 |
| X | Chemical Abstracts, Vol.128, abs.no.167044(1998) | 1 |
| P,X | JP 2005-126431 A (Semiconductor Energy Laboratory Co., Ltd.), 19 May, 2005 (19.05.05), Claim 1; Par. No. [0016] & US 2005/0079385 A1 | 1-3 |
| P,X | WO 2005/108348 A1 (Idemitsu Kosan Co., Ltd.), 17 November, 2005 (17.11.05), Page 43, lines 1 to 2; page 43, 3rd line from the bottom to page 44, line 5 (Family: none) | 1 |

Form PCT/ISA/210 (continuation of second sheet) (April 2005)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 5315078 A
- JP 2001118682 A